(19) Europäisches Patentamt
European Patent Office
Office européen des brevets

(11) Numéro de publication: **0 211 721**
**B1**

(12) **FASCICULE DE BREVET EUROPÉEN**

(45) Date de publication du fascicule du brevet: **04.10.89**

(21) Numéro de dépôt: **86401494.9**

(22) Date de dépôt: **04.07.86**

(51) Int. Cl.⁴: **C 07 C 91/28**, C 07 C 93/14, A 61 K 31/135

(54) **Phényléthanolaminotétralines, un procédé pour leur préparation et compositions pharmaceutiques en contenant.**

(30) Priorité: **10.07.85 FR 8510559**
**07.05.86 FR 8606626**

(43) Date de publication de la demande:
**25.02.87 Bulletin 87/9**

(45) Mention de la délivrance du brevet:
**04.10.89 Bulletin 89/40**

(84) Etats contractants désignés:
**AT BE CH DE FR GB IT LI LU NL SE**

(56) Documents cité:
**EP-A-0 033 789**
**DE-A-2 803 582**

**JOURNAL OF THE MEDICINAL CHEMISTRY, vol. 11, 1968, pages 1000-1008; R. HOWE et al.: "Beta-adrenergic blocking agents. I. Pronethalol and related N-alkyl and N-aralkyl derivatives of 2-amino-1-(2-naphthyl)ethanol"**

(73) Titulaire: **SANOFI S.A., 40, Avenue George V, F-75008 Paris (FR)**
(84) Etats contractants désignés:
**BE CH DE FR GB LI LU NL SE AT**

(73) Titulaire: **MIDY S.p.A., Société Anonyme dite:, Via Piranesi 38, I-20137 Milano (IT)**
(84) Etats contractants désignés: **IT**

(72) Inventeur: **Cecchi, Roberto, via Dei Tigli 1, I-20075 - Lodi Milan (IT)**
Inventeur: **Boigegrain, Robert, via Amundsen 5, I-20122 - Milan (IT)**
Inventeur: **Bianchetti, Alberto, via Corridoni 11, I-20122 - Milan (IT)**
Inventeur: **Poggesi, Elena, via Piranesi 43, I-20137 - Milan (IT)**
Inventeur: **Croci, Tiziano, via Giacinti 2 Rozzano, I-20089 - Milan (IT)**

(74) Mandataire: **Gillard, Marie- Louise, Cabinet Beau de Loménie 55, Rue d'Amsterdam, F-75008 Paris (FR)**

**EP 0 211 721 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

## Description

La présente invention concerne de nouvelles phényléthanolaminotétralines à activité lipolytique, un procédé pour leur préparation et des compositions pharmaceutiques les contenant en tant que principes actifs.

Il est connu (Nature, 281, 1979, 31-35) que le tissu adipeux brun (BAT, de l'anglais Brown Adipose Tissue) et le tissu adipeux blanc (WAT, de l'anglais White Adipose Tissue) jouent un rôle important dans la lipolyse et dans la thermogénèse; plus particulièrement, la thermogénèse aurait son origine métabolique dans le BAT.

Il a été également demontré (Int. J. Obesity, 8, 1984, 159-180) que certains types d'obésité sont associés à une capacité réduite de dissiper, comme chaleur, l'énergie provenant du régime alimentaire et que notamment le BAT peut être impliqué dans le contrôle de l'obésité, même si son rôle dans la thermogénèse causée par l'alimentation chez les rongeurs et chez l'homme reste à être établi sans équivoque.

On a maintenant trouvé que certaines phényléthanolaminotétralines et leurs sels possèdent une bonne activité stimulante sur le BAT et le WAT et qu'elles sont utiles dans le traitement de l'obésité.

On a déjà décrit des dérivés de la tétraline comme médicaments, comme notamment dans les brevets EP-A-033 789 et DE-2 803 582 et JOURNAL OF THE MEDICINAL CHEMISTRY vol. 11, 1968, p. 1000-1008. Toutefois aucun de ces documents ne décrit les phényléthanolaminotétralines selon l'invention. En effet, le EP-A-033 789 décrit des dérivés de la 2-amino-tétraline dans lesquels le groupe amino est substitué par un groupe aralkyle qui ne contient pas, dans le groupe alkyle, le groupe hydroxy caractéristique des phényléthanolamines. De plus, les composés décrits dans ce document ont une activité inotrope, donc cardiaque, qui est bien illustrée par des données chiffrées.

Le journal of the Medicinal Chemistry décrit, d'une part, des dérivés du pronéthalol, caractérisés donc par la structure naphtyléthanolamine et non phényléthanolamine et, d'autre part, des dérivés de l'aminotétraline non substitués dans le cycle tétralinique. Tous ces produits ont une activité bêta-bloquante, donc cardique. Aucune autre activité n'est indiquée dans ce document.

Le DE-A-2 803 582 décrit des aminotétralines substituées sur le groupe amino par l'hydrogène, un groupe alkyle, cycloalkyle ou aralkyle, ledit groupe aralkyle ne contenant pas, dans le groupe alkyle, le groupe hydroxy caractéristique des phényléthanolamines. De plus, les composés décrits dans ce document sont décrits en tant qu'agents stimulants les récepteurs alpha et bêta adrénergiques ainsi que les récepteurs de la dopamine. Ils sont indiqués comme agents actifs sur le système cardiovasculaire et sur le système nerveux central en tant qu'anti-parkinsoniens.

Ainsi la présente invention concerne, selon un de ses aspects, de nouvelles phényléthanolaminotétralines de formule:

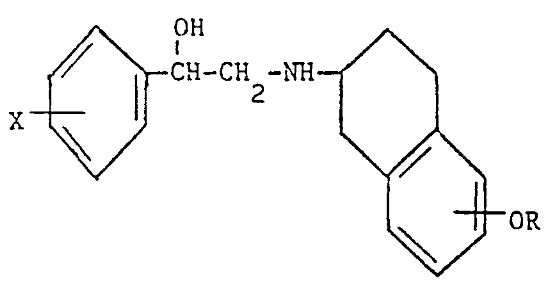

I

dans laquelle

X représente l'hydrogène, un halogène, un groupe trifluorométhyle ou un groupe alkyle inférieur et

R représente l'hydrogène ou un groupe méthyle non substitué ou substitué par un groupe carboxy ou carbalcoxy inférieur;

et leurs sels pharmaceutiquement acceptables.

Les composés de formule I ci-dessus peuvent être sous une forme optiquement inactive ou sous une forme optiquement active choisie parmi celles des énantiomères, des diastéréoisomères et de leurs mélanges. Tous ces composés et leurs sels pharmaceutiquement acceptables sont compris dans le cadre de la présente invention.

Le terme "alkyle inférieur", tel qu'utilisé ici, désigne un radical monovalent d'un hydrocarbure saturé contenant 1 à 4 atomes de carbone, tel que méthyle, éthyle, propyle, isopropyle ou n-butyle.

Les termes "alcoxy inférieur" et "carbalcoxy inférieur" désignent, respectivement, les groupes hydroxyles et carboxyle, respectivement éthérifié et estérifié avec un alkyle inférieur tel que défini ci-dessus.

Le terme "alcanoyle inférieur" désigne un groupe carbonyle substitué par un alkyle inférieur tel que défini ci-dessus.

Le terme "halogène" comprend les quatre halogènes fluor, chlore, brome, iode, les trois premiers étant particulièrement préférés.

Les composés de formule I et leurs sels pharmaceutiquement acceptables ont une activité lipolytique considérable et ils peuvent être utilisés pour le traitement de l'obésité.

Selon un autre de ses aspects, la présente invention concerne un procédé pour la préparation de composés de formule I et de leurs sels pharmaceutiquement acceptables.

Ledit procédé est caractérisé en ce qu'on fait réagir un composé de formule:

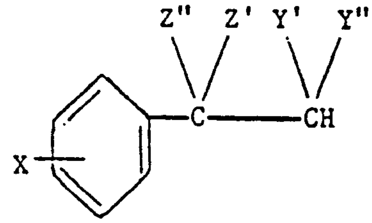

II

dans laquelle X est tel que défini ci-dessus; un des Z' et Z" est l'hydrogène et l'autre est un groupe hydroxy, ou bien Z' et Z", ensemble, forment un groupe oxo; un des Y' et Y" est l'hydrogène et l'autre est un halogène choisi parmi le chlore, le brome et l'iode ou un groupe amino, ou bien Y' et Y", ensemble, forment un groupe oxo; ou Y" et Z" sont tous les deux hydrogènes et Y' et Z', ensemble, forment un atome d'oxygène époxydique; avec une tétraline dont le substituant hydroxy éventuel est éventuellement progété, de formule:

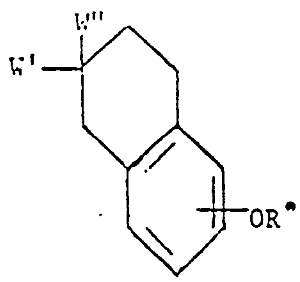

III

dans laquelle R° est hydrogène ou méthyleet soit un des W' et W" est l'hydrogène et l'autre est un groupe amino, soit W' et W", ensemble, forment un groupe oxo;

la réaction étant conduite, lorsque au moins un groupe oxo est présent dans un des composés II et III, en présence d'un agent réducteur;

on sépare éventuellement les stéréoisomères des composés obtenus;

on élimine les groupes protecteurs éventuellement présents;

lorsque le substituant R° du composé ainsi obtenu est l'hydrogène, on traite éventuellement ledit composé avec un halogénoacétate d'alkyle inférieur en présence d'hydrure de sodium;

et, après avoir éventuellement transformé le groupe carbalcoxy dans le groupe carboxyle correspondant par saponification, on transforme éventuellement le produit ainsi obtenu dans ses sels pharmaceutiquement acceptables;

avec la limitation que dans les composés II et III de départ:

- lorsque Y' ou Y" est un groupe amino, un des W' et W" est autre qu'un groupe amino,
- lorsque Y' et Y", ensemble, forment un groupe oxo, W' et W" sont, ensemble, autre qu'un groupe oxo, et
- lorsque Y' et Z', ensemble, forment un atome d'oxygène époxydique, alors W' et W" sont, ensemble, autre qu'un groupe oxo.

Dans la tétraline de départ de formule III, les groupes hydroxy et carboxy qui sont éventuellement présents peuvent être libres ou protégés.

Les groupes protecteurs du groupe hydroxyle comprennent un groupe acyle tel que, par exemple, formyle, acétyle, chloroacétyle, trifluoroacétyle, méthoxyacétyle, phénoxyacétyle, benzoyle, benzoylformyle, p-nitrobenzoyle, éthoxycarbonyle, bêta,bêta,bêta-trichloroéthoxycarbonyle, bêta,bêta,bêta-tribromoéthoxycarbonyle ou p-nitrophénoxycarbonyle, ou un groupe protecteur qui puisse être aisément éliminé dans des conditions d'hydrolyse relativement faibles tel que, par exemple, un groupe tétrahydropyranyle, tétrahydrothio furanyle ou méthoxytétrahydropyranyle ou un groupe protecteur qui puisse être éliminé par hydrogénation catalytique, tel que benzyle, benzhydryle, méthoxybenzyle, trityle.

Le groupe hydroxyle phénolique peut aussi être protégé par le groupe méthyle.

Le groupe hydroxyle protégé peyt être libéré selon les techniques conventionelles. Ainsi, le groupe méthoxyle aromatique peut être transformé dans le phénol correspondant selon les méthodes connues de déméthylation, par exemple par traitement avec les complexes de trifluorure ou tribromure de bore tel que le complexe tribromure de bore/diméthylsulfure. Les groupes benzyloxy, benzhydryloxy, méthoxybenzyloxy et trityloxy peuvent être libérés par hydrogénation catalytique.

Les groupes alcanoyloxy, tels que formyloxy et acétoxy, donnent l'hydroxyle libre correspondant par les méthodes connues de saponification.

Les groupes carbalcoxy, d'une manière analogue, sont transformés dans le groupe carboxyle correspondant par saponification.

Bien que la protection du groupe hydroxy de la tétraline de formule III ne soit pas nécessaire, elle peut être utile dans le cas d'une meilleure disponibilité de la matière première ou pour un meilleur déroulement de la réaction. Par exemple, la 6- et la 7-methoxy-2-tétralone sont plus disponibles que la 6- et la 7-hydroxy-2-tétralone.

Un mode opératoire préféré du procédé de la présente invention est caractérisé en ce qu'on fait réagir une phényléthanolamine racémique ou optiquement active de formule:

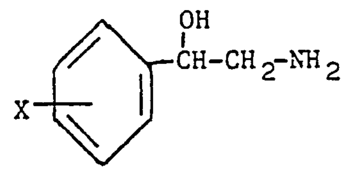

IIa

dans laquelle X est tel que défini ci-dessus, avec une tétralone dont le substituant hydroxy éventuel est éventuellement protégé, de formule:

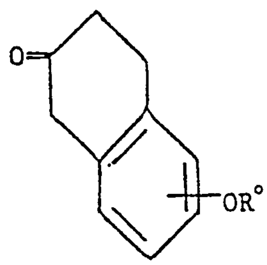

IIIa

dans laquelle R° est tel que défini ci-dessus, dans un solvant organique en présence d'un agent réducteur;

on sépare éventuellement les stéréoisomères des composés obtenus, avantageusement par cristallisation fractionnée dans un solvant approprié ou par chromatographie, puis on élimine les groupes protecteurs éventuellement présents;

lorsque le substituant R° du composé ainsi obtenu est l'hydrogène, on traite éventuellement ledit composé avec un halogénoacétate d'alkyle inférieur en présence d'hydrure de sodium;

et, après avoir éventuellement transformé le groupe carbalcoxy dans le groupe carboxyle correspondant par saponification, on transforme éventuellement le produit ainsi obtenu dans un de ses sels pharmaceutiquement acceptables.

Comme solvant organique préféré, on utilise un alcool aliphatique contenant de 1 à 6 atomes de carbone, tel que méthanol, éthanol, n-butanol, n-pentanol mais on peut utiliser d'autres solvants tels que hexane, benzène et toluène.

Selon cette méthode de préparation, qui comporte une amination réductrice, on opère en présence d'hydrogène et d'un catalyseur convenable tel que, par exemple, le bioxyde de platine, le Ni-Raney ou le palladium sur charbon, ou bien en présence d'un autre agent réducteur, par exemple un agent réducteur organique, tel que le cyanoborohydrure de sodium, le borohydrure de sodium, le cyanoborohydrure de tétrabutylammonium, le cyanoborohydrure de lithium ou le triéthylborohydrure de lithium.

La réaction est avantageusement conduite en présence d'un acide organique, tel que, par exemple, l'acide acétique glacial.

-La température de réaction peut varier entre la température ambiante (environ 20° C) et 50° C, préférablement entre 30 et 40° C environ et sa durée varie en conséquence. En général, après 3 à 6 heures de chauffage à 30 - 40° C, la réaction est complète et le produit final ainsi obtenu peut être isolé selon les techniques conventionelles.

Un autre mode opératoire avantageux du procédé de la présente invention est caractérisé en ce qu'on fait réagir un époxyde de formule:

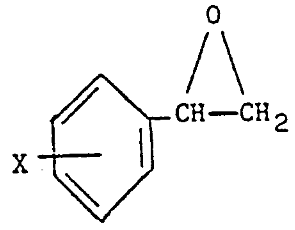

IIb

dans laquelle X est tel que défini ci-dessus, avec une aminotétraline, dont le substituant hydroxy éventuel est éventuellement protégé, de formule:

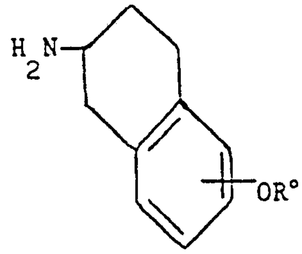

IIb

dans laquelle R° est tel que défini ci-dessus.

La réaction peut être conduite en absence de solvants ou en présence d'un solvant organique. En général, après 5 - 96 heures à une température de 0 à 50°C, la réaction est complète et le produit ainsi obtenu est isolé, éventuellement déprotégé; lorsque le substituant R° du composé ainsi obtenu est l'hydrogène, on traite éventuellement ledit composé avec un halogénoacetate d'alkyle inférieur en présence d'hydrure de sodium et, après avoir éventuellement transformé le groupe carbalcoxy dans le groupe carboxyle correspondant par saponification, on le transforme éventuellement dans un de ses sels.

Un autre mode opératoire avantageux du procédé de la présente invention est caractérisé en ce qu'on fait réagir un phénylglyoxal de formule:

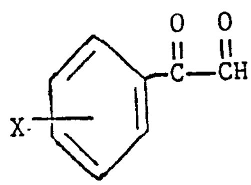

IIc

dans laquelle X est tel que défini ci-dessus, avec une aminotétraline, dont le substituant hydroxy éventuel est éventuellement protégé, de formule IIIb en présence d'un agent réducteur.

La réaction est conduite dans un solvant organique, en général à la température ambiante (environ 20°C).

Les agents réducteurs comprennent ceux décrits ci-dessus, par exemple le borohydrure de sodium.

Le produit obtenu est isolé, éventuellement déprotégé; lorsque le substituant R° du composé ainsi obtenu est l'hydrogène, on traite éventuellement ledit composé avec un halogénoacétate d'alkyle inférieur en présence d'hydrure de sodium et, après avoir éventuellement transformé le groupe carbalcoxy dans le groupe carboxyle correspondant par saponification, éventuellement on le transforme dans un de ses sels.

Un autre mode opératoire avantageux du procédé de la présente invention est caractérisé en ce qu'on fait réagir une alphahaloacétopénone de formule:

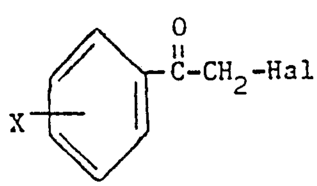

IId

dans laquelle X est tel que défini ci-dessus et Hal est choisi parmi le chlore, le brome et l'iode, avec une aminotétraline, dont le substituant hydroxy éventuel est éventuellement protégé, de formule IIIb en présence d'un agent réducteur.

Les conditions de réaction sont les mêmes décrites ci-dessus. Toutefois, comme un acide halogenhydrique se libère, il est avantageux de le neutraliser avec une amine tertiaire telle que la triéthylamine, d'isoler la cétone et de conduire la réduction sur le produit isolé. La phényléthanolaminotétraline ainsi obtenue peut être isolée, éventuellement déprotégée; lorsque le substituant R° du composé ainsi obtenu est l'hydrogène, on traite éventuellement ledit composé avec un halogénoacétate d'alkyle inférieur en présence d'hydrure de sodium et, après avoir éventuellement transformé le groupe carbalcoxy dans le groupe carboxyle correspondant par saponification, éventuellement on le transforme dans ses sels.

Une alpha-haloacétophénone de départ préférée de formule IId est l'alpha-bromoacétophénone.

Les composés de formule I présentent deux atomes de carbone asymétriques.

Selon un aspect ultérieur de la présente invention, les mélanges de diastéréoisomères sont obtenus par cristallisation dans un solvant approprié, de préférence un alcanol inférieur tel que l'isopropanol, l'éthanol ou leurs mélanges. On peut également passer desdits mélanges aux stéréoisomères selon les techniques connues, par exemple par séparation chromatographique ou par réaction avec un acide optiquement actif pour

former des sels séparables. Les acides optiquement actifs pour les procédés de résolution sont décrits dans Topics in Stereochemistry, vol. 6, Wiley Interscience 1971, Allinger N.L. and Eliel W.L. eds.

On peut également obtenir les stéréoisomères à partir d'un seul énantiomère d'une phényléthanolamine de formule IIa par réaction avec un composé de formule IIIa et réduction. Les diastéréoisomères ainsi obtenus peuvent être séparés par des techniques convenables comme la cristallisation fractionnée dans un solvant approprié, de préférence un alcanol inférieur tel que l'isopropanol, l'éthanol ou leurs mélanges.

On peut encore obtenir des stéréoisomères à partir d'un des énantiomères d'une aminotétraline de formule IIIb par réaction avec un composé de formule IIb, IIc ou IId et les isoler comme décrit ci-dessus.

On peut enfin obtenir des stéréoisomères purs à partir d'un des énantiomères d'un époxyde de formule IIb avec un des énantiomères d'une aminotétraline de formule IIIb.

Pour la nomenclature des racémates, ainsi que des stéréisomères et de leurs mélanges, on adopte ici la convention suivante:

- les produits obtenus à partir des racemates sont définis par leur nom chimique sans désignation de la stéréochimie;
- les mélanges de diastéréisomères obtenus à partir d'un des énantiomères soit d'un composé II soit d'un composé III sont respectivement définis "(1R,2'RS)", "(1S,2'RS)", "(1RS,2'R) et "(1RS,2'S);
- les stéréisomères dont un seul centre d'asymétrie a une configuration connue sont définis par le signe (+) ou (-) de la rotation optique et par la configuration du seul centre défini.

Les composés de formule I peuvent être transformés dans leurs sels pharmaceutiquement acceptables.

Lorsque le composé de formule I est sous forme de base libre, la salification est effectuée par traitement avec l'acide choisi dans un solvant organique.

Lorsque le composé de formule I possède un groupe carboxyle libre, son caractère amphotère permet la préparation de sels soit avec des acides soit avec des bases. Les sels avec des bases pharmaceutiquement acceptables sont de préférence ceux avec des métaux alcalins tels que le sodium, mais les sels avec des bases organiques, tels que le sel avec le trométamol, sont également convenables.

Par traitement de la base libre, dissoute par exemple dans un alcool tel que l'isopropanol, avec une solution de l'acide choisi dans le même solvant, on obtient le sel correspondant qui est isolé selon les techniques conventionnelles. Ainsi, on prépare par exemple le chlorhydrate, le bromhydrate, le sulfate, l'hydrogénosulfate, le dihydrogénophosphate, le méthanesulfonate, le méthylsulfate, l'oxalate, le maléate, le fumarate, le naphtalène-2-sulfonate.

A la fin des réactions, le composé de formule I peut être isolé sous forme d'un de ses sels, par exemple le chlorhydrate; dans ce cas, s'il est nécessaire, la base libre peut être préparée par neutralisation dudit sel avec une base minérale ou organique telle que l'hydroxyde de sodium ou la triéthylamine ou avec un carbonate ou bicarbonate alcalin, tel que le carbonate ou le bicarbonate de sodium ou de potassium.

Les composés de formule I et leur sels possèdent des propriétés pharmacologiques intéressantes. Plus particulièrement, les composés de la présente invention montrent une activité lipolitique considérable chez les mammifères dans les tests prédictifs de ladite activité tel que le test "in vitro" sur le tissu adipeux brun (BAT) et sur le tissu adipeux blanc (WAT) et le test "in vivo" fondé sur la thermogénèse chez le rat.

Plus particulièrement, dans le test "in vitro" on a sacrifié des rats mâles pesant chacun 100 - 125 grammes et on a prélévé les adipocytes du BAT interscapulaire et ceux du WAT epididymal selon la méthode de M. Rodbell, J. Biol. Chem. 242, 1967, 5744-5750 et on les a traités séparément de la façon suivante. On met en incubation, à 37°C pendant une heure, les adipocytes avec le produit à tester en milieu Phosphate Krebs-Ringer, contenant de l'albumine (4 %), à pH 7,4. Les acides gras à chaîne longue, libérés dans le milieu, sont déterminés selon la méthode de D.I. Trout et al., J. Lip. Res. 1, 1960, 199-202 et rapportés au contenu en triglycérides des adipocytes, mesuré selon la méthode de E. Van Handel et al., J. Lab. Clin. Med. 50, 1957, 152-157.

Les résultats obtenus pour des composés représentatifs de la présente invention sont consignés dans le Tableau I, où l'activité lipolytique des composés à la dose de $10^{-6}$ M, mesurée comme libération des acides gras libres (µEq), est exprimée en pourcentage de l'effet de la noradrénaline à la dose de $5 \cdot 10^{-6}$ M (effet maximal) sur le BAT et sur le WAT.

**Tableau I**

| Composé ($10^{-6}$ M) | Exemple N. | Activité lipolytique BAT | WAT |
|---|---|---|---|
| SR 58279 A | 1 | 50 % | 30 % |
| SR 58306 A | 4 | 80 % | 78 % |
| SR 58338 A | 7 | 77 % | 89 % |
| SR 58339 A | 8 | 103 % | 92 % |
| SR 58365 A | 18 | 96 % | 78 % |
| SR 58375 A | 22 | 100 % | 97 % |
| SR 58380 A | 11 | 96 % | 90 % |

Dans le test "in vivo", l'activité sur la thermogénèse du tissu adipeux brun est déterminée selon la méthode de P.J. Wellman, Research Communication in Chemical Pathology and Pharmacology 41, 1983, 173-176.

On traite les rats anesthésiés par voie intrapéritonéale par le véhicule et une dose de 7,5 mg/kg des substances à tester et on relève la température du BAT interscapulaire et la température rectale toutes les 10, 20 et 30 minutes après l'injection.

Le Tableau II montre les différences moyennes (ΔT) entre la température du BAT interscapulaire et la température rectale des animaux traités par un composé représentatif de la présente invention et des contrôles n'ayant reçu que le véhicule.

**Tableau II**

| Composé 7,5 mg/kg (par voie intrapéritonéale) | ΔT° C Temps 10' | 20' | 30' |
|---|---|---|---|
| Véhicule | 0,06 ± 0,04 | 0,10 ± 0,04 | 0,14 ± 0,05 |
| SR 58306 (Ex. 3) | 0,60 ± 0,10 | 0,90 ± 0,10 | 0,92 ± 0,08 |

Les résultats obtenus dans les tests "in vitro" et "in vivo" ci-dessus, montrent que les composés représentatifs de la présente invention ont une considérable activité lipolytique, aussi bien sur le BAT que sur le WAT et qu'ils activent d'une manière significative la thermogénèse dans le BAT de rats anesthésiés.

Les composés de formule I ci-dessus et leurs sels pharmaceutiquement acceptables sont aussi des modulateurs de la motricité intestinale et utérine comme démontré dans les tests du côlon et de l'utérus de rat et dans celui de la motricité gastrointestinale chez le chien.

En plus, les composés de la présente invention sont pratiquement inactifs comme stimulants bêta-1 ou bêta-2 adrénergiques comme démontré dans les tests classiques sur l'oreillette droite et sur la trachée isolées de cobaye.

Les composés de formule I ci-dessus et leurs sels pharmaceutiquement acceptables sont peu toxiques; leur DL50 chez la souris peut varier entre 15 et 35 mg/kg par voie intraveineuse et peut être supérieure à environ 350 mg/kg par voie orale; par exemple, les composés SR 58380 A (Exemple 11 ci-dessous) et SR 58339 A (Exemple 8 ci-dessous) ne donnent pas de mortalité aux souris jusqu'à la dose de 200 mg/kg par voie orale.

Ainsi, la présente invention, selon un autre de ses aspects, concerne des compositions pharmaceutiques utiles notamment pour le traitement de l'obésité, renfermant, en tant que principes actifs, les composés de formule I ci-dessus ainsi que leurs sels pharmaceutiquement acceptables.

Dans les compositions pharmaceutiques de la présente invention pour l'administration orale, sublinguale, sous-cutanée, intramusculaire, intraveineuse, transdermique ou rectale, les ingrédients actifs de formule I ci-dessus peuvent être administrés sous formes unitaires d'administration, en mélange avec des supports pharmaceutiques classiques, aux animaux et aux êtres humains pour le traitement de l'obésité. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire ou intraveineuse et les formes d'administration rectale.

Afin d'obtenir l'effet lipolytique désiré, la dose de principe actif peut varier entre 0,01 et 100 mg par kg de poids du corps et par jour.

Chaque dose unitaire peut contenir de 0,1 à 500 mg d'ingrédient actif en combinaison avec un support pharmaceutique. Cette dose unitaire peut être administrée 1 à 4 fois par jour.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir ou pour l'administration sous forme de gouttes peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les compositions de la présente invention peuvent contenir, à côté des produits de formule I ci-dessus ou d'un de leurs sels pharmaceutiquement acceptables, d'autres principes actifs tels que, par exemple, des anorexigènes, des tranquillisants, des antidépresseurs ou d'autres médicaments qui peuvent être utiles dans le traitement de l'obésité.

Les exemples suivants illustrent l'invention sans toutefois la limiter. Les valeurs de pouvoir rotatoire sont le résultat d'une seule détermination. Elles sont indiquées comme [alpha], mais on doit les entendre comme $[alpha]^{25}_D$.

**Exemple 1**

(a) On fait réagir à 35°C pendant 4 heures 1 g de 7-méthoxy-2-tétralone avec 0,8 g de 2-amino-1-phényléthanol dans 30 ml de méthanol et 1 ml d'acide acétique glacial en présence d'hydrogène et de 0,1 g de bioxyde de platine. Après filtration et concentration, on reprend le résidu par de l'eau alcalinisée avec de la soude concentrée et on extrait par de l'acétate d'éthyle. On sèche la solution sur du sulfate de sodium anhydre, on l'évapore à sec et on dissout le résidu dans de l'isopropanol. A la solution ainsi obtenue, on ajoute une solution saturée d'acide chlorhydrique gazeux dans l'isopropanol. On obtient ainsi le chlorhydrate de 2-[(7-méthoxy-1,2,3,4-tétrahydronapht-2-yl)amino]-1-phényléthanol, désigné par son numéro de code SR 58279 A, qui, après cristallisation dans l'isopropanol, fond à 185 - 187°C.

Rendement: 37 % de la théorie.

(b) On fait réagir sous agitation à la température ambiante pendant 4 jours 3,01 g de 2-amino-7-méthoxy-1,2,3,4-tétrahydronaphtalène avec 2,04 g d'oxyde de styrène. Après avoir ajouté de l'acétate d'éthyle, on ajoute au mélange réactionnel une solution saturée d'acide chlorhydrique gazeux dans l'isopropanol. On obtient ainsi le chlorhydrate de 2-[(7-méthoxy-1,2,3,4-tétrahydronapht-2-yl)amino]-1-phényléthanol.

Rendement: 31 % de la théorie.

(c) On fait réagir sous agitation à la température ambiante pendant 3 heures 3,53 g de phénylglyoxal avec 5 g du chlorhydrate de 2-amino-7-méthoxy-1,2,3,4-tétrahydronaphtalène dans 100 ml de méthanol en présence de 0,9 g de borohydrure de sodium. Après avoir refroidi à 4°C, on ajoute 4,3 g de borohydrure de sodium et on laisse le mélange réactionnel sous agitation à la température ambiante une nuit. Après avoir ajouté 20 ml d'eau, on agite à la température ambiante pendant 15 minutes et puis on évapore à sec la solution. On dissout le résidu dans 300 ml d'acétate d'éthyle et 60 m d'eau; après filtration, la phase organique est séparée, séchée sur du sulfate de magnésium, filtrée et évaporée à sec. On obtient une huile qui est purifiée par chromatographie éclair (flash-chromatography) en utilisant comme éluant un mélange d'acétate d'éthyle /méthanol 8/2. On obtient ainsi le chlorhydrate de 2-[(7-méthoxy-1,2,3,4-tétrahydronapht-2-yl)amino]-1-phényléthanol.

Rendement: 20 % de la théorie.

(d) A un mélange de 1,77 g de 2-amino-7-méthoxy-1,2,3,4-tétrahydronaphtalène et de 1,1 g de triéthylamine dans 25 ml de tétrahydrofuranne, on ajoute lentement 1,94 g de bromoacétophénone dans 50 ml de tétrahydrofuranne. Le mélange réactionnel est laissé sous agitation à la température ambiante pendant 2 heures. On filtre et on évapore. Le résidu ainsi obtenu est dissout dans l'isopropanol et acidifié avec une solution d'acide chlorhydrique gazeux dans l'isopropanol. Après précipitation par une solution éthérée, on filtre et on sèche. Au résidu dissout dans 70 ml de méthanol et refroidi à 4°C, on ajoute lentement 1,5 g de borohydrure de sodium. Après avoir mis sous agitation le mélange réactionnel pendant une heure à la température ambiante, on le verse dans l'eau et on l'acidifie par l'acide acétique. La solution est ensuite basifiée avec du bicarbonate de sodium et la phase organique est extraite avec le chlorure de méthylène, séchée et évaporée à sec. L'huile obtenue est chromatographiée en éluant avec un mélange d'acétate d'éthyle/méthanol 8/2. Après recristallisation dans l'isopropanol, on obtient le chlorhydrate de 2-[(7-méthoxy-1,2,3,4-tétrahydronapht-2-yl)amino]-1-phényléthanol.

Rendement: 30 % de la théorie.

**Exemple 2**

On fait réagir à 35°C pendant 3 heures 4 g de 6-méthoxy-2-tétralone avec 3,2 g de 2-amino-1-phényléthanol dans 100 ml de méthanol et 4 ml d'acide acétique glacial en présence d'hydrogène et de 0,3 g de bioxyde de platine. Après filtration et concentration, on reprend le résidu par de l'hydroxyde de sodium et on extrait par de l'acétate d'éthyle. On sèche la solution sur du sulfate de sodium anhydre et on l'évapore. On purifie l'huile noire ainsi obtenue par chromatographie éclair en utilisant comme éluant un mélange chlorure de methylène/méthanol 9/1. Les fractions contenant le produit sont réunies et évaporées à sec. On dissout le résidu dans 20 ml d'isopropanol et à la solution ainsi obtenue, on ajoute une solution saturée d'acide chlorhydrique gazeux dans l'isopropanol. On obtient ainsi le chlorhydrate de 2-[(6-méthoxy-1,2,3,4-tétrahydro-

napht-2-yl)amino]-1-phénylethanol, désigné par son numéro de code SR 58280 A, qui, après cristallisation dans l'isopropanol, fond à 174 - 176° C.

Rendement: 47,5 % de la théorie.

**Exemple 3**

On chauffe au reflux sous courant d'azote pendant 16 heures 3,3 g du chlorhydrate de 2-[(7-méthoxy-1,2,3,4-tétrahydronapht-2-yl)amino]-1-phényléthanol (SR 58279 A), obtenu comme décrit dans l'exemple 1, avec un mélange de 9,5 g du complexe bore tribromure/diméthylsulfure et de 100 ml de chlorure de méthylène anhydre. Après avoir refroidi la solution, on ajoute de l'acétate d'éthyle et de l'eau alcalinisée avec une solution saturée de bicarbonate sodique. On extrait la solution par de l'acétate d'éthyle et, après l'avoir séchée sur du sulfate de magnésium, on la filtre et on évapore à sec. On purifie le solide pâteux ainsi obtenu par chromatographie éclair en éluant avec un mélange chloroforme/méthanol 8/2. Après deux cristallisations dans 7 ml d'acétate d'éthyle, on obtient le 2-[(7-hydroxy-1,2,3,4-tétrahydronapht-2-yl)amino]-1-phényléthanol, désigné par son numéro de code SR 58306; p.f. 156 - 158° C.

Rendement: 60 % de la théorie.

Le produit contient les deux diastéréoisomères (1R,2'R;1S,2'S) dans un rapport d'environ 1/1 (déterminé par RMN à 250 MHz).

**Exemple 4**

A une solution dans l'isopropanol de 1,7 g de 2-[(7-hydroxy-1,2,3,4-tétrahydronapht-2-yl)amino]-1-phényléthanol, obtenu comme décrit dans l'exemple 3, on ajoute de l'acide chlorhydrique gazeux dans l'isopropanol. On obtient, après précipitation par l'éther éthylique, le chlorhydrate de 2-[(7-hydroxy-1,2,3,4-tétrahydronapht-2-yl)amino]-1-phényléthanol, désigné par son numéro de code SR 58306 A, qui, après séchage, fond à 157 - 159° C.

Rendement: 56 % de la théorie.

**Exemple 5**

On fait réagir sous agitation pendant 30 minutes à la température ambiante 3,9 g de 2-[(7-hydroxy-1,2,3,4-tétrahydronapht-2-yl)amino]-1-phényléthanol, obtenu comme décrit dans l'exemple 3, dans 40 ml de diméthylsulfoxyde et en présence de 0,63 g d'hydrure de sodium dispersé dans de la huile (55 %). Après addition de 2,1 ml de bromoacétate d'éthyle (0,0137 mole), on laisse le mélange réactionnel à la température ambiante pendant 3 heures et 30 minutes, puis on le verse dans 300 ml d'eau et on l'extrait avec 300 ml d'acétate d'éthyle. On sèche, filtre, évapore à sec et on obtient une huile qui est purifiée par chromatographie éclair en éluant avec un mélange acétate d'éthyle/méthanol 95/5. On laisse réagir pendant une nuit l'huile purifiée avec un excès d'acide oxalique dans 5 ml d'isopropanol. On obtient ainsi l'oxalate de 2-[(7-carbéthoxyméthoxy-1,2,3,4-tétrahydronapht-2-yl)amino]-1 phényléthanol, désigné par son numéro de code SR 58436 A; p.f. 159 - 162° C.

Rendement : 5 % de la théorie.

**Exemple 6**

On chauffe au reflux sous courant d'azote pendant 16 heures 1,5 g du chlorhydrate de 2-[(6-méthoxy-1,2,3,4-tétrahydronapht-2-yl)amino]-1-phényléthanol, obtenu comme décrit dans l'exemple 2, dans une solution de 4,3 g du complexe bore tribromure/diméthylsulfure dans 80 ml de chlorure de méthylène anhydre. Après avoir refroidi la solution, on ajoute de l'acétate d'éthyle et de l'eau alcalinisée avec une solution saturée de bicarbonate sodique. On extrait la solution par de l'acétate d'éthyle et, après l'avoir séchée sur du sulfate de magnésium, on la filtre et on l'évapore à sec. On dissout le produit ainsi obtenu dans l'isopropanol et on acidifie avec de l'acide chlorhydrique gazeux dans l'isopropanol. On précipite le produit en ajoutant de l'éther éthylique et l'on obtient ainsi le chlorhydrate de 2-[(6-hydroxy-1,2,3,4-tétrahydronapht-2-yl)amino]-1-phényléthanol, désigné par son numéro de code SR 58307 A, qui, après séchage, fond à 166 - 168° C.

Rendement: 35% de la théorie.

**Exemple 7**

On fait réagir sous agitation à la température ambiante pendant 3 heures 4,8 g de 2-amino-1-(3-chlorophényl)-éthanol avec 5 g de 7-méthoxy-2-tétralone dans 80 ml de méthanol et 5 ml d'acide acétique glacial. On ajoute au mélange réactionnel 2,7 g de cyanoborohydrure de sodium et on laisse la solution ainsi obtenue sous agitation pendant une nuit. On acidifie, on évapore à sec, on reprend le résidu par 40 ml d'eau alcalinisée et on extrait par 200 ml d'acétate d'éthyle. On sèche la phase organique ainsi obtenue sur du sulfate de sodium anhydre, on filtre et on évapore à sec. On obtient une huile qui est purifiée par chromatographie éclair en éluant avec un mélange acétate d'éthyle/méthanol 95/5. On ajoute à la solution ainsi obtenue 40 ml d'isopropanol et 150 ml d'éther éthylique, puis quelques gouttes d'isopropanol saturé d'acide chlorhydrique gazeux. On obtient ainsi le chlorhydrate de 2-[(7-méthoxy-1,2,3,4-tétrahydronapht-2-yl)amino]-1-(3-chlorophényl)éthanol, désigné par son numéro de code SR 58338 A, qui, après cristallisation dans l'isopropanol, fond à 186 - 188°C.

Rendement: 40 % de la théorie.

**Exemple 8**

A une suspension de 3,7 g du chlorhydrate de 2-[(7-méthoxy-1,2,3,4-tétrahydronapht-2-yl)amino]-1-(3-chlorophényl)éthanol, obtenu comme décrit dans l'exemple 7, dans 60 ml de chlorure de méthylène anhydre, on ajoute goutte à goutte, sous courant d'azote et à la température de -20°C, 60 ml d'une solution 1 M de tribromure de bore dans le chlorure de méthylène. On laisse le mélange réactionnel sous agitation à la température de -20°C pendant 3 heures et 30 minutes. On ajoute un excès de glace, on alcalinise avec de l'ammoniaque concentrée et on sépare la phase organique qui est séchée sur du sulfate de sodium anhydre et évaporée à sec. Le résidu ainsi obtenu est purifié par chromatographie éclair en éluant avec un mélange chlorure de méthylène/méthanol 9/1. Au 2-[(7-hydroxy-1,2,3,4-tétrahydronapht-2-yl)amino]-1-(3-chlorophényl)éthanol ainsi obtenu sous forme de base, dissous dans un mélange de 10 ml d'isopropanol et de 30 ml d'éther éthylique, on ajoute une solution saturée d'acide chlorhydrique gazeux en isopropanol; on obtient ainsi le chlorhydrate de 2-[(7-hydroxy-1,2,3,4-tétrahydronapht-2-yl)amino]-1-(3-chlorophényl)éthanol, désigné par son numéro de code SR 58339 A, qui, après cristallisation dans 20 ml d'éthanol absolu, fond à 113 - 115°C.

Rendement: 30 % de la théorie.

**Exemple 9**

En ajoutant à la base obtenue comme décrit dans l'exemple 8 une solution à 48 % d'acide bromhydrique dans l'eau, on obtient le bromhydrate de 2-[(7-hydroxy-1,2,3,4-tétrahydronapht-2-yl)amino]-1-(3-chlorophényl)éthanol, désigné par son numéro de code SR 58339 B; p.f. 126 - 128°C.

Rendement: 65 % de la théorie.

**Exemple 10**

En ajoutant à la base obtenue comme décrit dans l'exemple 8 une solution d'acide fumarique dans l'eau, on obtient le fumarate de 2-[(7-hydroxy-1,2,3,4-tétrahydronapht-2-yl)amino]-1-(3-chlorophényl)éthanol, désigné par son numéro de code SR 58339 C; p.f. 223 - 225°C.

Rendement: 56 % de la théorie.

**Exemple 11**

On laisse sous agitation à la température ambiante pendant 30 minutes une solution dans 50 ml de diméthylsulfoxyde de 7,1 g du chlorhydrate de 2-[(7-hydroxy-1,2,3,4-tétrahydronapht-2-yl)amino]-1-(3-chlorophényl)éthanol, obtenu comme décrit dans l'exemple 8, en présence de 1,9 g d'une dispersion d'hydrure de sodium à 55 % dans l'huile (FLUKA). On ajoute au mélange réactionnel 3,3 g de bromoacétate d'éthyle et on le laisse sous agitation à la température ambiante pendant 24 heures. On verse la solution ainsi obtenue dans 300 ml d'eau, on extrait avec 300 ml d'acétate d'éthyle, on sèche la phase organique sur du sulfate de sodium, on filtre et on évapore à sec. L'huile ainsi obtenue est purifiée par chromatographie éclair en éluant avec un mélange acétate d'éthyle/méthanol 95/5. On laisse réagir pendant 36 heures l'huile purifiée avec un excès d'acide oxalique dans 20 ml d'isopropanol et on obtient ainsi l'oxalate de 2-[(7-carbéthoxyméthoxy-1,2,3,4-tétrahydronapht-2-yl)amino]-1-(3-chlorophényl)éthanol, désigné par son numéro de code SR 58380 A; p.f. 145 - 147°C.
Rendement: 5 % de la théorie.

**Exemple 12**

On laisse réagir sous agitation à la température ambiante pendant 3 heures 8,5 g de D(-)phényléthanolamine (J. Org. Chem. 45, 1980, 2785) et 12 g de 7-méthoxy-2-tétralone dans 150 ml de méthanol en présence de 9 ml d'acide acétique glacial. On laisse le mélange réactionnel sous agitation à la température ambiante pendant une nuit en présence de 5,8 g de cyanoborohydrure de sodium. On acidifie à pH 3 avec de l'acide chlorhydrique concentré et on laisse le mélange réactionnel sous agitation à la température ambiante pendant 30 minutes. On évapore à sec, on reprend le résidu avec 80 ml d'eau et on alcalinise avec de l'ammoniaque concentrée. On extrait par 400 ml d'acétate d'éthyle et on lave la phase organique ainsi obtenue avec 40 ml d'eau. On sèche sur du sulfate de sodium anhydre, on filtre et on évapore à sec. Le produit ainsi obtenu est purifié par chromatographie éclair en éluant avec un mélange acétate d'éthyle/méthanol 9/1. On dissout le produit dans 70 ml d'isopropanol, on ajoute à la solution ainsi obtenue une solution saturée d'acide chlorhydrique gazeux dans l'isopropanol. On obtient ainsi le chlorhydrate de (1R,2'RS)-2-[(7-méthoxy-1,2,3,4-tétrahydronapht-2-yl)amino]-1-phényléthanol, désigné par son numéro de code SR 58361 A, p.f. 195 - 199°C, [alpha] = -30° (eau/éthanol 1/1, c = 2 %).
Rendement: 43,5 % de la théorie.
Le produit contient les deux diastéréoisomères (1R,2'R) et (1R,2'S) dans un rapport d'environ 1/1 (déterminé par HPLC).

**Exemple 13**

Selon le mode opératoire de l'exemple 12, à partir de 14 g de L(+)phényléthanolamine (J. Org. Chem. 45, 1980, 2785) et 19,8 g de 7-méthoxy-2-tétralone, on obtient le chlorhydrate de (1S,2'RS )-2-[(7-méthoxy-1,2,3,4-tétrahydronapht-2-yl)amino]-1-phényléthanol, désigné par son numéro de code SR 58358 A, p.f. 194 - 198°C, [alpha] +28° (eau/éthanol 1/1, c = 2 %).
Rendement: 47,6 % de la théorie.
Le produit contient les deux diastéréoisomères (1S,2'R) et (1S,2'S) dans un rapport d'environ 1/1 (déterminé par HPLC).

**Exemple 14**

Six grammes de chlorhydrate de (1R,2'RS)-2-/77-méthoxy-1,2,3,4-tétrahydronapht-2-yl)-amino]-1-phényléthanol obtenu comme décrit dans l'exemple 12, sont cristallisés quatre fois de suite dans un mélange isopropanol/éthanol à 95 % 6/4. On réunit les eaux mères des cristallisations et on évapore à sec. Le produit obtenu est cristallisé dans 40 ml d'éthanol absolu. Les eaux mères de cette dernière cristallisation sont évaporées et leur résidu trituré dans 20 ml d'éther éthylique. On obtient ainsi le chlorhydrate de (-)-(1R)-2-[(7-méthoxy-1,2,3,4-tétrahydronapht-2-yl)amino]-1-phényléthanol, désigné par son numéro de code SR 58362 A, p.f. 169 - 171°C, [alpha] = -92° (eau/éthanol 1/1, c = 2 %).
Rendement: 25 % de la théorie.

**Exemple 15**

Selon le mode opératoire de l'exemple 14, à partir de 11 g de chlorhydrate de (1S,2'RS)-2-[(7-méthoxy-1,2,3,4-tétrahydronapht-2-yl)amino]-1-phényléthanol, obtenu comme décrit dans l'exemple 13, on obtient le chlorhydrate de (+)-(1S)-2-[(7-méthoxy-1,2,3,4-tétrahydronapht-2-yl)amino]-1-phényléthanol, désigné par son numéro de code SR 58359 A, p.f. 172 - 175°C, [alpha] = +95° (eau/éthanol 1/1, c = 2 %).
Rendement: 20 % de la théorie.

**Exemple 16**

Six grammes de chlorhydrate de (1R,2'RS)-2-[(7-méthoxy-1,2,3,4-tétrahydronapht-2-yl)-amino]-1-phényléthanol, obtenu comme décrit dans l'exemple 12, sont cristallisés six fois de suite dans un mélange

isopropanol/éthanol à 95 % 6/4. On obtient le chlorhydrate de (+)-(1R)-2-[(7-méthoxy-1,2,3,4-tétrahydronapht-2-yl)amino]-1-phényléthanol, désigné par son numéro de code SR 58363 A, p.f. 220 - 222°C, [alpha] = +32° (eau/éthanol 1/1, c = 2 %).

Rendement: 30 % de la théorie.

**Exemple 17**

Selon le mode opératoire de l'exemple 16, à partir de 11 g de chlorhydrate de (1S,2'RS)-2-[(7-méthoxy-1,2,3,4-tétrahydronapht-2-yl)amino]-1-phényléthanol, obtenu comme décrit dans l'exemple 13, on obtient le chlorhydrate de (-)-(1S)-2-[(7-méthoxy-1,2,3,4-tétrahydronapht-2-yl)amino]-1-phényléthanol, désigné par son numéro de code SR 58360 A, p.f. 219 - 221°C, [alpha] = -35° (eau/éthanol 1/1, c = 2 %).

Rendement: 20 % de la théorie.

**Exemple 18**

A 45 ml d'une solution 1 M de tribromure de bore dans du chlorure de méthylène, on ajoute, goutte à goutte, sous courant d'azote, à la température de -20°C et pendant 15 minutes, 2,5 g d'une suspension du chlorhydrate de (1R,2'RS)-2-[(7-méthoxy-1,2,3,4-tétrahydronapht-2-yl)amino]-1-phényléthanol, obtenu comme décrit dans l'exemple 12, dans 80 ml de chlorure de méthylène anhydre. On laisse le mélange réactionnel sous agitation à la température de -20°C pendant 3 heures et 30 minutes, puis on y ajoute 150 ml de glace et on l'alcalinise avec de l'ammoniaque concentrée. On laisse le mélange réactionnel sous agitation à la température ambiante pendant 30 minutes et ensuite on extrait par 300 ml d'acétate d'éthyle. La phase organique est séparée, séchée, filtrée et évaporée à sec. L'huile obtenue est purifiée par chromatographie éclair en éluant avec un mélange acétate d'éthyle/méthanol 9/1. Au produit ainsi obtenu, dissous dans l'isopropanol, on ajoute de l'isopropanol contenant de l'éther éthylique saturé d'acide chlorhydrique gazeux; on évapore le solvant et on triture le résidu avec de l'éther éthylique. On obtient ainsi le chlorhydrate de (1R,2'RS)-2-[(7-hydroxy-1,2,3,4-tétrahydronapht-2-yl)amino]-1-phényléthanol, désigné par son numéro de code SR 58365 A, p.f. 150 - 152°C, [alpha] = -35° (eau/éthanol 1/1, c = 2 %).

Rendement: 37% de la théorie.

**Exemple 19**

Selon le mode opératoire de l'exemple 18, à partir de 2,5 g de chlorhydrate de (1S,2'RS)-2-[(7-méthoxy-1,2,3,4-tétrahydronapht-2-yl)amino]-1-phényléthanol, obtenu comme décrit dans l'exemple 13, on obtient le chlorhydrate de (1S,2'RS)-2-[(7-hydroxy-1,2,3,4-tétrahydronapht-2-yl)amino]-1-phényléthanol, désigné par son numéro de code SR 58364 A, p.f. 153 - 155°C, [alpha] = +37° (eau/éthanol 1/1, c = 2 %).

Rendement: 7 % de la théorie.

**Exemple 20**

A 27 ml d'une solution 1 M de tribromure de bore dans du chlorure de méthylène, on ajoute, goutte à goutte, sous courant d'azote, à la température de -18°C et pendant 10 minutes, 1,5 g d'une suspension du chlorhydrate de (-)-(1R)-2-[(7-méthoxy-1,2,3,4-tétrahydronapht-2-yl)amino]-1-phényléthanol, obtenu comme décrit dans l'exemple 14, dans 40 ml de chlorure de méthylène anhydre. On laisse le mélange réactionnel sous agitation à la température de -18°C pendant 6 heures et on y ajoute ensuite 100 ml de glace. On l'alcalinise avec de l'ammoniaque concentrée, on extrait par 200 ml d'acétate d'éthyle et on sépare la phase organique qui est séchée, filtrée et évaporée à sec. L'huile ainsi obtenue est purifiée par chromatographie éclair en éluant avec un mélange acétate d'éthyle/méthanol 9/1. A une solution du produit dans 10 ml d'isopropanol, on ajoute une solution saturée d'acide chlorhydrique gazeux en isopropanol et ensuite 60 ml d'éther éthylique. On obtient ainsi le chlorhydrate de (-)-(1R)-2-[(7-hydroxy-1,2,3,4-tétrahydronapht-2-yl)-amino]-1-phényléthanol, désigné par son numéro de code SR 58374 A, p.f. 205 - 207°C, [alpha] = -91° (eau/éthanol 1/1, c = 2 %).

Rendement: 17 % de la théorie.

**Exemple 21**

Selon le mode opératoire de l'exemple 20, à partir de 3,2 g de chlorhydrate de (+)-(1S)-2-[(7-méthoxy-1,2,3,4-tétrahydronapht-2-yl)-amino]-1-phényléthanol, obtenu comme décrit dans l'exemple 15, on obtient le chlorhydrate de (+)-(1S)-2-[(7-hydroxy-1,2,3,4-tétrahydronapht-2-yl)-amino]-1-phényléthanol, désigné par son numéro de code SR 58372 A, p.f. 200 - 202°C, [alpha] = +89° (eau/éthanol 1/1, c = 2 %).
Rendement: 30 % de la théorie.

**Exemple 22**

A 23,4 ml d'une solution 1 M de tribromure de bore dans du chlorure de méthylène, on ajoute, goutte à goutte, sous courant d'azote, à la température de -18°C et pendant 10 minutes, 1,3 g d'une suspension du chlorhydrate de (+)-(1R)-2-[(7-méthoxy-1,2,3,4-tétrahydronapht-2-yl)amino]-1-phényléthanol, obtenu comme décrit dans l'exemple 16, dans le chlorure de méthylène anhydre. On laisse le mélange réactionnel sous agitation à la température de -18°C pendant 3 heures et 30 minutes et on ajoute ensuite 100 ml de glace. On alcalinise avec de l'ammoniaque concentrée, on extrait par 250 ml d'acétate d'éthyle et on sépare la phase organique qui est séchée, filtrée et évaporée à sec. L'huile ainsi obtenue est purifiée par chromatographie éclair en éluant avec un mélange acétate d'éthyle/méthanol 9/1. A une solution du produit ainsi obtenu dans l'isopropanol, on ajoute de l'isopropanol saturé d'acide chlorhydrique gazeux. On évapore à sec et on triture le résidu avec 60 ml d'éther éthylique. On obtient ainsi le chlorhydrate de (+)-(1R)-2-[(7-hydroxy-1,2,3,4-tétrahydronapht-2-yl)amino]-1-phényléthanol, désigné par son numéro de code SR 58375 A, p.f. 175 - 177°C, [alpha] = +30° (eau/éthanol 1/1, c = 2 %).
Rendement: 50 % de la théorie.

**Exemple 23**

Selon le mode opératoire de l'exemple 22, à partir de 2 g de chlorhydrate de (-)-(1S)-2-[(7-méthoxy-1,2,3,4-tétrahydronapht-2-yl)amino]-1-phényléthanol, obtenu comme décrit dans l'exemple 17, on obtient le chlorhydrate de (-)-(1S)-2-[(7-hydroxy-1,2,3,4-tétrahydronapht-2-yl)amino]-1-phényléthanol, désigné par son numéro de code SR 58373 A, p.f. 177 - 179°C, [alpha] = -33° (eau/éthanol 1/1, c = 2 %).
Rendement: 30 % de la théorie.

**Exemple 24**

On prépare des gélules à base d'un des composés des exemples 1 à 23, ayant la composition suivante:

| | |
|---|---|
| principe actif | 15 mg |
| lactose | 120 mg |
| stéarate de magnesium | 5 mg |

en mélangeant intimement les ingrédients ci-dessus et en versant le mélange dans des gélules de gélatine dure.

**Exemple 25**

On prépare des comprimés à base d'un des composés des exemples 1 à 23 ayant la composition suivante:

| | |
|---|---|
| principe actif | 20 mg |
| lactose | 100 mg |
| cellulose microcristalline | 30 mg |
| amidon de mais séché | 40 mg |
| stéarate de magnesium | 5 mg |

en broyant l'ingrédient actif jusqu'à une dimension particulaire de 0,4 mm, en le faisant passer à travers un tamis de 0,4 mm d'ouverture de maille, en mélangeant la matière broyée avec les autres constituants et en comprimant pour former les comprimés.
De la même façon, on prépare des comprimés contenant 40 mg d'ingrédient actif.

**Exemple 26**

En opérant comme décrit dans l'exemple 25 ci-dessus, on prépare des comprimés ayant la composition suivante:

| | |
|---|---|
| principe actif | 50 mg |
| lactose | 95 mg |
| amidon de mais séché | 100 mg |
| talc | 4,5 mg |
| stéarate de magnesium | 0,5 mg |

**Exemple 27**

10.000 gélules avec un teneur en substance active de 50 mg sont préparées à partir des constituants suivants: 500 g de chlorhydrate de 2-[(7-methoxy-1,2,3,4-tétrahydronapht-2-yl)amino]-1-phényléthanol (SR 58279 A), 495 g de cellulose microcristalline, 5 g de gel de silice amorphe. Les constituants ci-dessus sont bien mélangés et introduits dans des gélules de gélatine dure de dimension 4.

**Exemple 28**

On prépare une solution aqueuse stérile appropriée pour une utilisation parentérale en ampoules à base de chlorhydrate de 2-[(7-hydroxy-1,2,3,4-tétrahydronapht-2-yl)amino]-1-phényléthanol (SR 58306 A) ayant la composition suivante:

| | |
|---|---|
| SR 58306 A | 30 mg |
| chlorure de sodium | 5 mg |
| eau distillée q.s.p. | 2 ml |

**Exemple 29**

On prépare des suppositoires à base d'un des composés des exemples 1 à 23, ayant la composition suivante:

| | |
|---|---|
| principe actif | 50 mg |
| lactose | 250 mg |
| Witespol W 45 q.s.p. | 1,7 g |

On mélange la substance active avec le lactose et on met le mélange uniformement en suspension dans la masse pour suppositoires fondue. On verse la suspension dans des moules refroidis pour former des suppositoires d'un poids de 1,7 g.

**Exemple 30**

On prépare des comprimés à base d'un des composes des exemples 1 à 23 ayant la composition suivante:

| | |
|---|---|
| principe actif | 25 mg |
| lactose | 95 mg |
| amidon de mais séché | 45 mg |
| silice colloïdale | 2 mg |
| amidon soluble | 5 mg |
| stéarate de magnésium | 3 mg |

On mélange la substance active avec une partie des adjuvants et on granule le mélange avec une solution d'amidon soluble dans l'eau. Après séchage du granulat, on ajoute le reste des adjuvants et on forme des tablettes par compression.

**Revendications** pour les Etats Contractants: BE, CH, DE, FR, GB, IT, LI, LU, NL, SE

1. Composé de formule

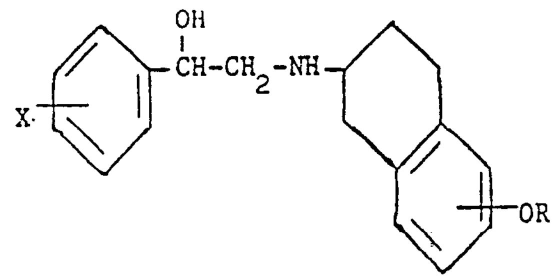

I

dans laquelle X représente l'hydrogène, un halogène, un groupe trifluorométhyle ou un groupe alkyle en $C_1$-$C_4$ et R représente l'hydrogène ou un groupe méthyle non substitué ou substitué par un groupe carboxy ou carbalcoxy dont le groupe alkyle est en $C_1$-$C_4$ ou un de ses sels pharmaceutiquement acceptables.

2. Composé de formule I selon la revendication 1, caractérisé en ce qu'il est choisi parmi:
le 2-[(7-hydroxy-1,2,3,4-tétrahydronapht-2-yl)amino]-1-phényléthanol,
le 2-[(7-hydroxy-1,2,3,4-tétrahydronapht-2-yl)amino]-1-(3-chlorophényl)éthanol,
le 2-[(7-carbéthoxyméthoxy-1,2,3,4-tétrahydronapht-2-yl)amino]-1-(3-chlorophényl)éthanol,
le 2-[(7-carbéthoxyméthoxy-1,2,3,4-tétrahydronapht-2-yl)amino]-1-phényléthanol ou un de leurs sels pharmaceutiquement acceptables.

3. Composé selon l'une des revendications 1 ou 2 sous forme d'un stéréoisomère unique ou sous forme d'un mélange de stéréoisomères.

4. Composé de formule I selon la revendication 1, caractérisé en ce qu'il est choisi parmi:
le (1R,2'RS)-2-[(7-hydroxy-1,2,3,4-tétrahydronapht-2-yl)amino]-1-phényléthanol,
le (1S,2'RS)-2-[(7-hydroxy-1,2,3,4-tétrahydronapht-2-yl)amino]-1-phényléthanol,
le (+)-(1R)-2-[(7-hydroxy-1,2,3,4-tétrahydronapht-2-yl)amino]-1-phényléthanol,
le (+)-(1S)-2-[(7-hydroxy-1,2,3,4-tétrahydronapht-2-yl)amino]1-phényléthanol,
le (-)-(1R)-2-[(7-hydroxy-1,2,3,4-tétrahydronapht-2-yl)amino]-1-phényléthanol,
le (-)-(1S)-2-[(7-hydroxy-1,2,3,4-tétrahydronapht-2-yl)amino]-1-phényléthanol ou un de leurs sels pharmaceutiquement acceptables.

5. Procédé pour la préparation d'un composé selon l'une quelconque des revendications 1 à 4, caractérisé en ce qu'on fait réagir un composé de formule:

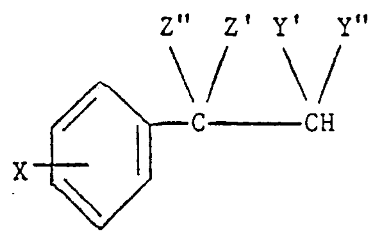

II

dans laquelle X est tel que défini ci-dessus; un des Z' et Z" est l'hydrogène et l'autre est un groupe hydroxy, ou bien Z' et Z", ensemble, forment un groupe oxo; un des Y' et Y" est l'hydrogène et l'autre est un halogène choisi parmi le chlore, le brome et l'iode ou un groupe amino, ou bien Y' et Y", ensemble, forment un groupe oxo; ou Y" et Z" sont tous les deux hydrogène et Y' et Z', ensemble, forment un atome d'oxygène époxydique; avec une tétraline, dont le substituant hydroxy éventuel est éventuellement protégé, de formule:

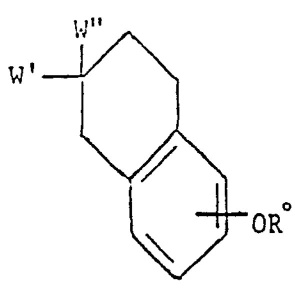

III

dans laquelle R° est hydrogène ou méthyle et soit un des W' et W" est l'hydrogène et l'autre est un groupe amino, soit W' et W", ensemble, forment un groupe oxo, la réaction étant conduite, lorsque au moins un

groupe oxo est présent dans un des composés II et III, en présence d'un agent réducteur; on sépare éventuellement les stéréoisomères des composés obtenus; on élimine les groupes protecteurs éventuellement présents; lorsque le substituant R° du composé ainsi obtenu est l'hydrogène, on traite éventuellement ledit composé avec un halogénoacétate d'alkyle en $C_1$-$C_4$, en présence d'hydrure de sodium; et après avoir éventuellement transformé le groupe carbalcoxy dans le groupe carboxyle correspondant par saponification, on transforme éventuellement le produit ainsi obtenu dans ses sels pharmaceutiquement acceptables; avec la limitation que dans les composés de départ II et III:

- lorsque Y' ou Y" est un groupe amino, un des W' et W" est autre qu'un groupe amino,
- lorsque Y' et Y", ensemble, forment un groupe oxo, W' et W" sont, ensemble, autre qu'un groupe oxo, et
- lorsque Y' et Z', ensemble, forment un atome d'oxygène époxydique, alors W' et W" sont, ensemble, autre qu'un groupe oxo.

6. Procédé selon la revendication 5 , caractérisé en ce qu'on fait réagir une phényléthanolamine racémique ou optiquement active de formule:

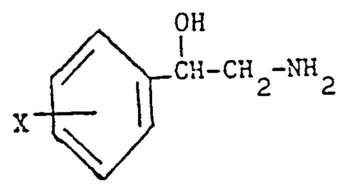

IIa

dans laquelle X est tel que défini ci-dessus, avec une tétralone, dont le substituant hydroxy éventuel est éventuellement protégé, de formule:

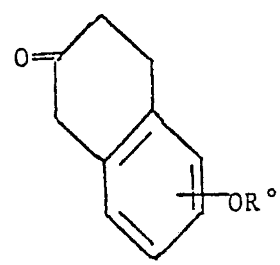

IIIa

dans laquelle R° est tel que défini ci-dessus, dans un solvant organique en présence d'un agent réducteur; on sépare éventuellement les stéréoisomères des composés obtenus, puis on élimine les groupes protecteurs éventuellement présents; lorsque le substituant R° du composé ainsi obtenu est l'hydrogène, on traite éventuellement ledit composé avec un halogénoacétate d'alkyle en $C_1$-$C_4$ en présence d'hydrure de sodium; et, après avoir éventuellement transformé le groupe carbalcoxy dans le groupe carboxyle correspondant par saponification, on transforme éventuellement le produit ainsi obtenu dans un de ses sels pharmaceutiquement acceptables.

7. Procédé selon la revendication 5, caractérisé en ce qu'on fait réagir un composé époxydique de formule:

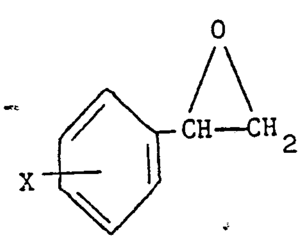

IIb

dans laquelle X est tel que défini ci-dessus, avec une aminotétraline, dont le substituant hydroxy éventuel est éventuellement protégé, de formule:

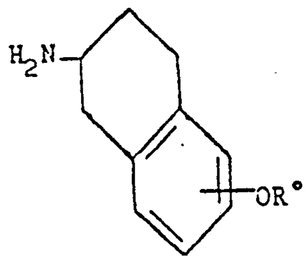

IIIb

dans laquelle R° est tel que défini ci-dessus, on sépare éventuellement les stéréoisomères des composés obtenus, puis on élimine les groupes protecteurs éventuellement présents; lorsque le substituant R° du composé ainsi obtenu est l'hydrogène, on traite éventuellement ledit composé avec un halogénoacétate d'alkyle en $C_1$-$C_4$ en présence d'hydrure de sodium; et, après avoir éventuellement transformé le groupe carbalcoxy dans le groupe carboxyle correspondant par saponification, on transforme éventuellement le produit ainsi obtenu dans un de ses sels pharmaceutiquement acceptables.

8. Procédé selon la revendication 5, caractérisé en ce qu'on fait réagir un phénylglyoxal de formule:

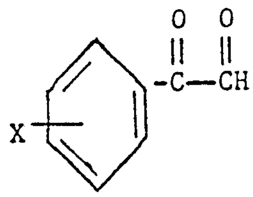

IIc

dans laquelle X est tel que défini ci-dessus, avec une aminotétraline, dont le substituant hydroxy éventuel est éventuellement protégé, de formule IIIb telle que définie dans la revendication 7, en présence d'un agent réducteur, on sépare éventuellement les stéréoisomères des composés obtenus, puis on élimine les groupes protecteurs éventuellement présents; lorsque le substituant R° du composé ainsi obtenu est l'hydrogène, on traite éventuellement ledit composé avec un halogénoacétate d'alkyle en $C_1$-$C_4$ en présence d'hydrure de sodium; et, après avoir éventuellement transformé le groupe carbalcoxy dans le groupe carboxyle correspondant par saponification, on transforme éventuellement le produit ainsi obtenu dans un de ses sels pharmaceutiquement acceptables.

9. Procédé selon la revendication 5, caractérisé en ce qu'on fait réagir une alpha-acétophénone de formule:

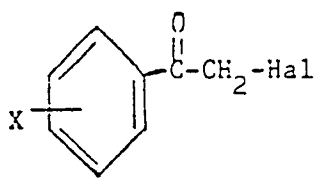

IId

dans laquelle X est tel que défini ci-dessus et Hal est choisi parmi le chlore, le brome et l'iode, avec une aminotétraline, dont le substituant hydroxy éventuel est éventuellement protégé, de formule IIIb, telle que définie dans la revendication 7, en présence d'un agent réducteur, on sépare éventuellement les stéréoisomères des composés obtenus, puis on élimine les groupes protecteurs éventuellement présents, lorsque le substituant R° du composé ainsi obtenu est l'hydrogène, on traite éventuellement ledit composé avec un halogénoacétate d'alkyle en $C_1$-$C_4$ en présence d'hydrure de sodium; et, après avoir éventuellement transformé le groupe carbalcoxy dans le groupe carboxyle correspondant par saponification, on transforme éventuellement le produit ainsi obtenu dans un de ses sels pharmaceutiquement acceptables.

10. Procédé selon la revendication 9, caractérisé en ce que la réaction est conduite aussi en présence d'une amine tertiaire.

11. Procédé selon l'une quelconque des revendications 5, 9 ou 10, caractérisé en ce que l'agent réducteur est ajouté après la neutralisation et l'isolement de la cétone obtenue.

12. Procédé selon l'une quelconque des revendications 5 à 11, caractérisé en ce que la séparation des stéréoisomères de formule I est effectuée par cristallisation fractionnée dans un solvant approprié ou par chromatographie.

13. Procédé selon la revendication 12, caractérisé en ce que le solvant approprié est un mélange d'isopropanol et d'éthanol.

14. Procédé selon l'une quelconque des revendications 5, 6, 8 à 13, caractérisé en ce que l'agent réducteur est l'hydrogène en présence d'un catalyseur d'hydrogénation.

15. Procédé selon l'une quelconque des revendications 5, 6, 8 à 13, caractérisé en ce que l'agent réducteur est un complexe d'hydrure métallique.

16. Procédé selon la revendication 15, caractérisé en ce que le complexe d'hydrure métallique est le complexe cyano-borohydrure de sodium.

17. Procédé selon l'une quelconque des revendications 5 à 16, caractérisé en ce qu'on isole le produit final sous forme d'un de ses sels et qu'on transforme ledit sel dans la base libre correspondante.

18. Composition pharmaceutique contenant, en tant que principe actif, un composé selon l'une quelconque des revendications 1 à 4.

19. Composition pharmaceutique selon la revendication 18 sous forme d'unité de dosage, dans laquelle le principe actif est melangé à un excipient pharmaceutique.

20. Composition pharmaceutique selon l'une des revendications 18 ou 19, contenant de 0,1 à 500 mg de principe actif.

**Revendications** pour l'Etat Contractant: AT

1. Procédé pour la préparation de nouvelles phényléthanolaminotétralines de formule:

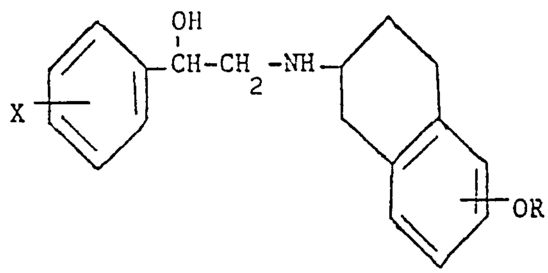

I

dans laquelle X représente l'hydrogène, un halogène, un groupe trifluorométhyle ou un groupe alkyle en $C_1$-$C_4$ et R représente l'hydrogène ou un groupe méthyle non substitué ou substitué par un groupe carboxy ou carbalcoxy dont le groupe alkyle est en $C_1$-$C_4$ ou de leurs sels pharmaceutiquement acceptables, caractérisé en ce qu'on fait réagir un composé de formule:

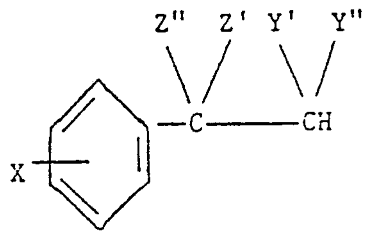

II

dans laquelle X est tel que défini ci-dessus; un des Z' et Z" est l'hydrogène et l'autre est un groupe hydroxy, ou bien Z' et Z", ensemble, forment un groupe oxo; un des Y' et Y" est l'hydrogène et l'autre est un halogène choisi parmi le chlore, le brome et l'iode ou un groupe amino, ou bien Y' et Y", ensemble, forment un groupe oxo; ou Y" et Z" sont tous les deux hydrogène et Y' et Z', ensemble, forment un atome d'oxygène époxydique; avec une tétraline, dont les substituants hydroxy et carboxy éventuels sont éventuellement protégés, de formule:

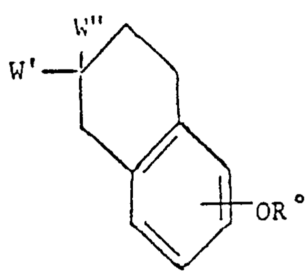

III

dans laquelle R° est hydrogène ou méthyle et soit un des W' et W" est l'hydrogène et l'autre est un groupe amino, soit W' et W", ensemble, forment un groupe oxo, la réaction étant conduite, lorsque au moins un groupe oxo est présent dans un des composés II et III, en présence d'un agent réducteur; on sépare éventuellement les stéréoisomères des composés de formule I; on élimine les groupes protecteurs éventuellement présents; lorsque le substituant R du composé ainsi obtenu est l'hydrogène, on traite éventuellement ledit composé avec un halogénoacétate d'alkyle en $C_1$-$C_4$ en présence d'hydrure de sodium; et, après avoir éventuellement transformé le groupe carbalcoxy dans le groupe carboxyle correspondant par

saponification, on transforme éventuellement le produit ainsi obtenu en un de ses sels pharmaceutiquement acceptables; avec la limitation que dans les composés de départ II et III:

- lorsque Y' ou Y" est un groupe amino, un des W' et W" est autre qu'un groupe amino,
- lorsque Y' et Y", ensemble, forment un groupe oxo, W' et W" sont, ensemble, autre qu'un groupe oxo, et
- lorsque Y' et Z', ensemble, forment un atome d'oxygène époxydique, alors W' et W" sont, ensemble, autre qu'un groupe oxo.

2. Procédé selon la revendication 1, caractérisé en ce qu'on fait réagir une phényléthanolamine racémique ou optiquement active de formule:

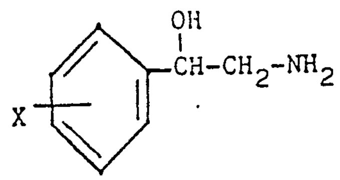

IIa

dans laquelle X est tel que défini ci-dessus, avec une tétralone, dont le substituant hydroxy éventuel est éventuellement protégé, de formule:

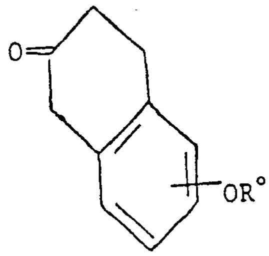

IIIa

dans laquelle R° est tel que défini ci-dessus, dans un solvant organique en présence d'un agent réducteur; on sépare éventuellement les stéréoisomères des composés obtenus, puis on élimine les groupes protecteurs éventuellement présents; lorsque le substituant R° du composé ainsi obtenu est l'hydrogène, on traite éventuellement ledit composé avec un halogénoacétate d'alkyle en $C_1$-$C_4$ en présence d'hydrure de sodium; et, après avoir éventuellement transformé le groupe carbalcoxy dans le groupe carboxyle correspondant par saponification, on transforme éventuellement le produit ainsi obtenu dans un de ses sels pharmaceutiquement acceptables.

3. Procédé selon la revendication 1, caractérisé en ce qu'on fait réagir un composé époxydique de formule:

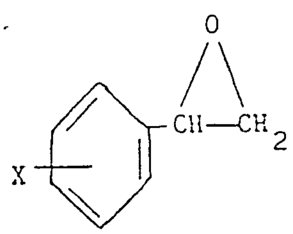

IIb

dans laquelle X est tel que défini ci-dessus, avec une aminotétraline, dont le substituant hydroxy éventuel est éventuellement protégé, de formule:

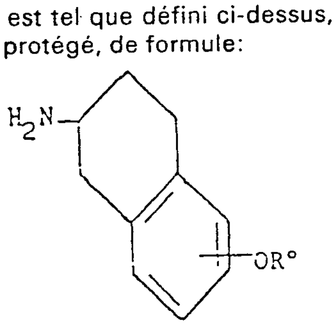

IIIb

dans laquelle R° est tel que défini ci-dessus, on sépare éventuellement les stéréoisomers des composés obtenus, puis on élimine les groupes protecteurs éventuellement présents; lorsque le substituant R° du composé ainsi obtenu est l'hydrogène, on traite éventuellement ledit composé avec un halogénoacétate d'alkyle en $C_1$-$C_4$ en présence d'hydrure de sodium; et, après avoir éventuellement transformé le groupe carbalcoxy dans le groupe carboxyle correspondant par saponification, on transforme éventuellement le produit ainsi obtenu dans un de ses sels pharmaceutiquement acceptables.

4. Procédé selon la revendication 1, caractérisé en ce qu'on fait réagir un phénylglyoxal formule:

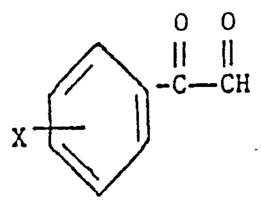

IIc

dans laquelle X est tel que défini ci-dessus, avec une aminotétraline, dont le substituant hydroxy éventuel est éventuellement protégé, de formule IIIb telle que définie dans la revendication 3, en présence d'un agent réducteur, on sépare éventuellement les stéréoisomères des composés obtenus, puis on élimine les groupes protecteurs éventuellement présents; lorsque le substituant R° du composé ainsi obtenu est l'hydrogène, on traite éventuellement ledit composé avec un halogénoacétate d'alkyle en $C_1$-$C_4$ en présence d'hydrure de sodium; et, après avoir éventuellement transformé le groupe carbalcoxy dans le groupe carboxyle correspondant par saponification, on transforme éventuellement le produit ainsi obtenu dans un de ses sels pharmaceutiquement acceptables.

5. Procédé selon la revendication 1, caractérisé en ce qu'on fait réagir une alpha-acétophénone de formule:

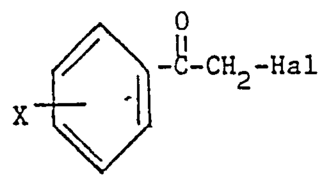

IId

dans laquelle X est tel que défini ci-dessus et Hal est choisi parmi le chlore, le brome et l'iode, avec une aminotétraline, dont le substituant hydroxy éventuel est éventuellement protégé, de formule IIIb, telle que définie dans la revendication 3, en présence d'un agent réducteur, on sépare éventuellement les stéréoisomères des composés obtenus, puis on élimine les groupes protecteurs éventuellement présents; lorsque le substituant R° du composé ainsi obtenu est l'hydrogène, on traite éventuellement ledit composé avec un halogénoacétate d'alkyle en $C_1$-$C_4$ en présence d'hydrure de sodium; et, après avoir éventuellement transformé le groupe carbalcoxy dans le groupe carboxyle correspondant par saponification, on transforme éventuellement le produit ainsi obtenu dans un de ses sels pharmaceutiquement acceptables.

6. Procédé selon la revendication 5, caractérisé en ce que la réaction est conduite aussi en présence d'une amine tertiaire.

7. Procédé selon l'une quelconque des revendications 1, 5 ou 6 caractérisé en ce que l'agent réducteur est ajouté après la neutralisation et l'isolement de la cétone obtenue.

8. Procédé selon l'une quelconque des revendications 1 à 6, caractérisé en ce que la séparation des stéréoisomères de formule I est effectuée par cristallisation fractionnée dans un solvant approprié ou par chromatographie.

9. Procédé selon la revendication 8, caractérisé en ce que le solvant approprié est un mélange d'isopropanol et d'éthanol.

10. Procédé selon l'une quelconque des revendications 1, 2 ou 4 à 9, caractérisé en ce que l'agent réducteur est l'hydrogène en présence d'un catalyseur d'hydrogénation.

11. Procédé selon l'une quelconque des revendications 1, 2 ou 4 à 9, caractérisé en ce que l'agent réducteur est un complexe d'hydrure métallique.

12. Procédé selon la revendication 11, caractérisé en ce que le complexe d'hydrure métallique est le complexe cyanoborohydrure de sodium.

13. Procédé selon l'une quelconque des revendications 1 à 12, caractérisé en ce qu'on isole le produit final sous forme d'un de ses sels et qu'on transforme ledit sel dans la base libre correspondante.

14. Procédé selon l'une quelconque des revendications précédentes, caractérisé en ce que l'on isole le composé de formule I sous forme d'un stéréoisomère unique ou sous forme d'un mélange de stéréoisomères.

15. Procédé selon l'une quelconque des revendications 1 à 14, caractérisé en ce que l'on prépare un composé de formule I choisi parmi le 2-[(7-hydroxy-1,2,3,4-tétrahydronapht-2-yl)amino]-1-phényléthanol
le 2-[(7-hydroxy-1,2,3,4-tétrahydronapht-2-yl)amino]-1-(3-chlorophényl)éthanol
le 2-[(7-carbéthoxyméthoxy-1,2,3,4-tétrahydronapht-2-yl)amino]-1-(3-chlorophényl)éthanol,
le 2-[(7-carbéthoxyméthoxy-1,2,3,4-tétrahydronapht-2-yl)amino]-1-phényléthanol ou un de leurs sels pharmaceutiquement acceptables.

16. Procédé selon l'une quelconque des revendications 1 à 14, caractérisé en ce que l'on prépare un composé de formule I choisi parmi le (1R,2'RS)-2-[(7-hydroxy-1,2,3,4-tétrahydronapht-2-yl)amino]-1-phényléthanol,
le (1S,2'RS)-2-[(7-hydroxy-1,2,3,4-tétrahydronapht-2-yl)amino]-1-phényléthanol,
le (+)-(1R)-2-[(7-hydroxy-1,2,3,4-tétrahydronapht-2-yl)amino]-1-phényléthanol,
le (+)-(1S)-2-[(7-hydroxy-1,2,3,4-tétrahydronapht-2-yl)amino]-1-phényléthanol,

le (-)-(1R)-2-[(7-hydroxy-1,2,3,4-tétrahydronapht-2-yl)amino]-1-phényléthanol,
le (-)-(1S)-2-[(7-hydroxy-1,2,3,4-tétrahydronapht-2-yl)amino]-1-phényléthanol ou un de leurs sels pharmaceutiquement acceptables.

**Claims** for the Contracting States: BE, CH, DE, FR, GB, IT, LI, LU, NL, SE

1. Compound of formula:

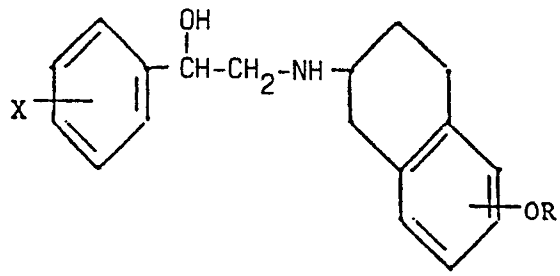

I

wherein X represents hydrogen, halogen, a trifluoromethyl or a $C_1$-$C_4$ alkyl group and R represents hydrogen or a methyl group unsubstituted or substituted by a carboxy or carbalkoxy group whose alkyl group is a $C_1$-$C_4$ alkyl or one of its pharmaceutically acceptable salts.

2. Compound of formula I according to claim 1, characterized in that it is selected from:
the 2-[(7-hydroxy-1,2,3,4-tetrahydronaphth-2-yl)amino]-1-phenylethanol,
the 2-[(7-hydroxy-1,2,3,4-tetrahydronaphth-2-yl)amino]-1-(3-chlorophenyl)ethanol
the 2-[(7-carbethoxymethoxy-1,2,3,4-tetrahydronaphth-2-yl)amino]-1-(3-chlorophenyl)ethanol,
the 2-[(7-carbethoxymethoxy-1,2,3,4-tetrahydronaphth-2-yl)amino]-1-phenylethanol or one of their pharmaceutically acceptable salts.

3. Compound according to any one of claims 1 or 2 in the form of a single stereoisomer or in the form of a mixture of stereoisomers.

4. Compound of formula I according to claim 1, characterized in that it is selected from:
(1R,2'RS)-2-[(7-hydroxy-1,2,3,4-tetrahydronaphth-2-yl)amino]-1-phenylethanol,
(1S,2'RS)-2-[(7-hydroxy-1,2,3,4-tetrahydronaphth-2-yl)amino]-1-phenylethanol,
the (+)-(1R)-2-[(7-hydroxy-1,2,3,4-tetrahydronaphth-2-yl)amino]-1-phenylethanol,
the (+)-(1S)-2-[(7-hydroxy-1,2,3,4-tetrahydronaphth-2-yl)amino]-1-phenylethanol,
the (-)-(1R)-2-[(7-hydroxy-1,2,3,4-tetrahydronaphth-2-yl)amino]-1-phenylethanol,
the (-)-(1S)-2- [(7-hydroxy-1,2,3,4-tetrahydronaphth-2-yl)amino]-1-phenylethanol or one of their pharmaceutically acceptable salts.

5. Process for the preparation of a compound according to any one of the claims 1 to 4, characterized in that it comprises reacting a compound of formula:

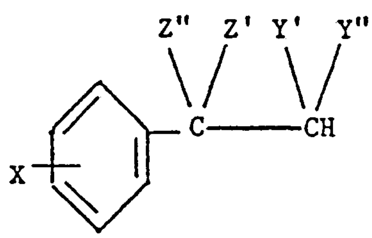

II

wherein X is as defined above; one of Z' and Z" is hydrogen and the other is a hydroxy group or Z' and Z", together, form an oxo group; one of Y' and Y" is hydrogen and the other is a halogen selected from chlorine, bromine and iodine or an amino group, or Y' and Y", together, form an oxo group; or, both Y" and Z" are both hydrogen and Y' and Z', together, form an epoxy oxygen atom; with a tetraline, having its optional hydroxy substituent optionally protected, of formula:

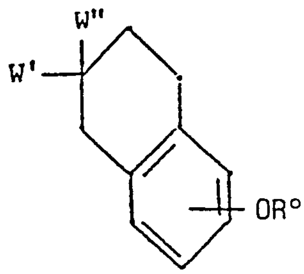

III

wherein R° is hydrogen or methyl and either one of W' and W" is hydrogen and the other is an amino group or W' and W", together, form an oxo group, the reaction being carried out, when at least one oxo group is present in one of the compounds II and III, in the presence of a reducing agent; optionally separating the stereoisomers of the obtained compounds; eliminating the optionally present protecting groups; when the substituent R° of the compound thus obtained is hydrogen, optionally treating the said compound with a $C_1$-$C_4$ alkyl halogenoacetate, in the presence of sodium hydride; and after having optionally converted the carbalkoxy group in the corresponding carboxyl group by saponification, optionally converting the product thus obtained into its pharmaceutically acceptable salts; with the proviso that in the reacting compounds II and III:

- when Y' or Y" is an amino group, one of W' and W" is other than an amino group,
- when Y' and Y", together, form an oxo group, W' and W" are, together, other than oxo group, and
- when Y' and Z', together, form an epoxy oxygen atom, then W' and W", together, are other than oxo group.

6. Process according to claim 5, characterized in that it comprises reacting a racemic or optically active phenylethanolamine of formula:

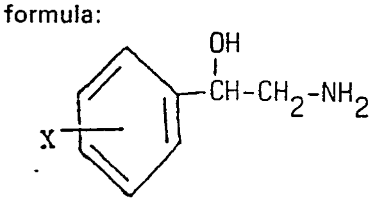

IIa

wherein X is as defined above, with a tetralone, having its optional hydroxy substituent optionally protected, of formula:

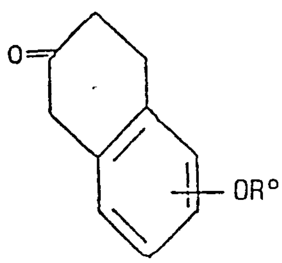

IIIa

wherein R° is as defined above, in an organic solvent in the presence of a reducing agent; optionally separating the stereoisomers of the obtained compounds, then eliminating the optionally present protecting groups; when the substituent R° of the compound thus obtained is hydrogen, optionally treating the said compound with a $C_1$-$C_4$ alkyl halogenoacetate, in the presence of sodium hydride; and after having optionally converted the carbalkoxy group in the corresponding carboxyl group by saponification, optionally converting the product thus obtained into one of its pharmaceutically acceptable salts.

7. Process according to claim 5, characterized in that it comprises reacting an epoxy compound of formula:

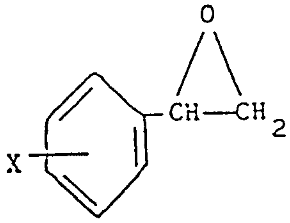

IIb

wherein X is as defined above, with an aminotetraline, having its optional hydroxy substituent optionally protected, of formula:

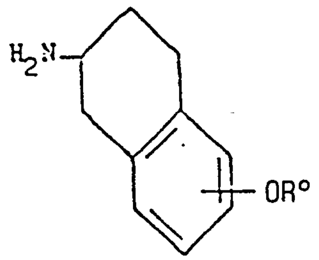

IIIb

wherein R° is as defined above; optionally separating the stereoisomers of the obtained compounds, then eliminating the optionally present protecting groups; when the substituent R° of the compound thus obtained is hydrogen, optionally treating the said compound with a $C_1$-$C_4$ alkyl halogenoacetate in the presence of sodium hydride; and after having optionally converted the carbalkoxy group in the corresponding carboxyl group by saponification, optionally converting the product thus obtained into one of its pharmaceutically acceptable salts.

8. Process according to claim 5, characterized in that it comprises reacting a phenylglyoxal of formula:

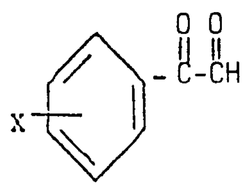

IIc

wherein X is as defined above, with an aminotetraline, having its optional hydroxy substituent optionally protected, of formula IIIb as defined in claim 7, in the presence of a reducing agent, optionally separating the stereoisomers of the obtained compounds, then eliminating the optionally present protecting groups; when the substituent R° of the compound thus obtained is hydrogen, optionally treating the said compound with a $C_1$-$C_4$ alkyl halogenoacetate, in the presence of sodium hydride; and after having optionally converted the carbalkoxy group in the corresponding carboxyl group by saponification, optionally converting the product thus obtained into one of its pharmaceutically acceptable salts.

9. Process according to claim 5, characterized in that it comprises reacting an alpha-acetophenone of formula:

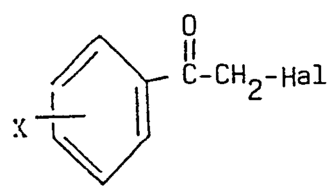

IId

wherein X is as defined above and Hal is selected from chlorine, bromine and iodine, with an aminotetraline, having its optional hydroxy substituent optionally protected, of formula IIIb as defined in claim 7, in the presence of a reducing agent, optionally separating the stereoisomers of the obtained compounds then eliminating the optionally present protecting groups; when the substituent R° of the compound thus obtained is hydrogen, optionally treating the said compound with a $C_1$-$C_4$ alkyl halogenoacetate, in the presence of sodium hydride; and after having optionally converted the carbalkoxy group in the corresponding carboxyl group by saponification, optionally converting the product thus obtained into one of its pharmaceutically acceptable salts.

10. Process according to claim 9, characterized in that the reaction is carried out also in the presence of a tertiary amine.

11. Process according to any one of claims 5, 9 or 10, characterized in that the reducing agent is added after the neutralization and the isolation of the obtained ketone.

12. Process according to any one of claims 5 to 11, characterized in that the separation of stereoisomers of compounds of formula I is carried out by fractional crystallization in an appropriate solvent or by chromatography.

13. Process according to claim 12, characterized in that the appropriate solvent is a mixture of isopropyl alcohol and ethanol.

14. Process according to any one of claims 5, 6 and 8 to 13, characterized in that the reducing agent is hydrogen in the presence of a hydrogenation catalyst.

15. Process according to any one of claims 5, 6 and 8 to 13, characterized in that the reducing agent is a metal hydride complex.

16. Process according to claim 15, characterized in that the metal hydride complex is the sodium cyanoborohydride complex.

17. Process according to any one of claims 5 to 16, characterized in that it comprises isolating the final product in the form of one of its salts thereof and converting said salt into the corresponding free base.

18. Pharmaceutical composition comprising as active ingredient a compound according to any one of claims 1 to 4.

19. Pharmaceutical composition according to claim 18 in dosage unit form, in which the active ingredient is in admixture with a pharmaceutical carrier.

20. Pharmaceutical composition according to any one of claims 18 to 19 comprising from 0.1 to 500 mg of active ingredient.

**Claims** for the Contracting State: AT

1. Process for the preparation of novel phenylethanolaminotetralines of formula:

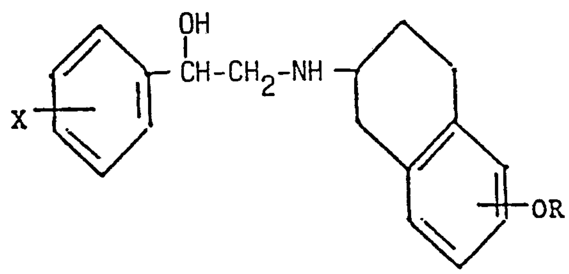

I

wherein X represents hydrogen, halogen, a trifluoromethyl or a $C_1$-$C_4$ alkyl group and R represents hydrogen or a methyl group unsubstituted or substituted by a carboxy or carbalkoxy group whose alkyl group is a $C_1$-$C_4$ alkyl or of their pharmaceutically acceptable salts characterized in that it comprises reacting a compound of formula:

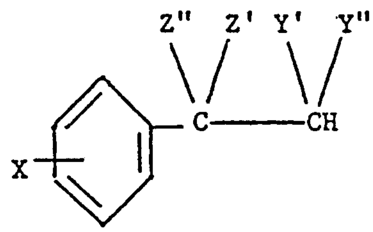

II

wherein X is as defined above; one of Z' and Z" is hydrogen and the other is a hydroxy group or Z' and Z", together, form an oxo group; one of Y' and Y" is hydrogen and the other is a halogen selected from the chlorine, bromine and iodine or an amino group; or Y' and Y", together, form an oxo group; or, both Y" and Z" are hydrogen and Y' and Z', together, form an epoxy oxygen atom ; with a tetraline, having its optional hydroxy and carboxy substituents optionally protected, of formula:

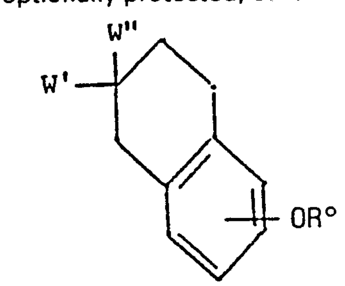

III

wherein R° is hydrogen or methyl and either one of W' and W" is hydrogen and the other is an amino group or W' and W", together, form an oxo group, the reaction being carried out, when at least one oxo group is present in one of the compounds II and III, in the presence of a reducing agent; then optionally separating the stereoisomers of the compounds of formula I; eliminating the optionally present protecting groups; when the substituent R° of the compound thus obtained is hydrogen, optionally treating the said compound with a $C_1$-$C_4$ alkyl halogenoacetate, in the presence of sodium hydride; and after having optionally converted the carbalkoxy group in the corresponding carboxyl group by saponification, optionally converting the product thus obtained into one of its pharmaceutically acceptable salts; with the proviso that in the reacting

compounds II and III:
- when Y' or Y" is an amino group, one of W' and W" is other than an amino group,
- when Y' and Y", together, form an oxo group, then W' and W" are, together, other than oxo group, and
- when Y' and Z', together, form an epoxy oxygen atom, then W' and W", together, are other than oxo group.

2. Process according to claim 1, characterized in that it comprises reacting a racemic or optically active phenylethanolamine of formula:

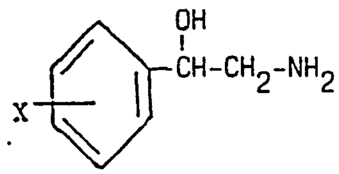

IIa

wherein X is as defined above, with a tetralone, having its optional hydroxy substituent optionally protected, of formula:

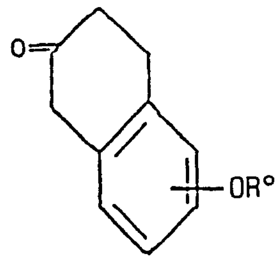

IIIa

wherein R° is as defined above, in an organic solvent in the presence of a reducing agent; optionally separating the stereoisomers of the obtained compounds, then eliminating the optionally present protecting groups; when the substituent R° of the compound thus obtained is hydrogen, optionally treating the said compound with a $C_1$-$C_4$ alkyl halogenoacetate, in the presence of sodium hydride; and after having optionally converted the carbalkoxy group in the corresponding carboxyl group by saponification, optionally converting the product thus obtained into one of its pharmaceutically acceptable salts.

3. Process according to claim 1, characterized in that it comprises reacting an epoxy compound of formula:

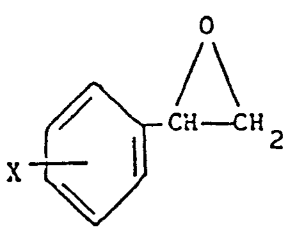

IIb

wherein X is as defined above, with an aminotetraline, having its optional hydroxy substituent optionally protected, of formula:

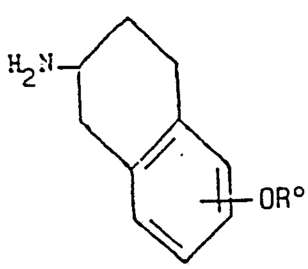

IIIb

wherein R° is as defined above; optionally separating the stereoisomers of the obtained compounds, then eliminating the optionally present protecting groups; when the substituent R° of the compound thus obtained is hydrogen, optionally treating the said compound with a $C_1$-$C_4$ alkyl halogenoacetate in the presence of sodium hydride; and after having optionally converted the carbalkoxy group in the corresponding carboxyl group by saponification, optionally converting the product thus obtained into one of its pharmaceutically acceptable salts.

4. Process according to claim 1, characterized in that reacting a phenylglyoxal of formula:

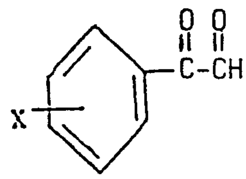

IIc

wherein X is as defined above, with an aminotetraline, having its optional hydroxy substituent optionally protected, of formula IIIb as defined in claim 3, in the presence of a reducing agent, optionally separating the stereoisomers of the obtained compounds, then eliminating the optionally present protecting groups; when the substituent R° of the compound thus obtained is hydrogen, optionally treating the said compound with a $C_1$-$C_4$ alkyl halogenoacetate, in the presence of sodium hydride; and after having optionally converted the carbalkoxy group in the corresponding carboxyl group by saponification, optionally converting the product thus obtained into one of its pharmaceutically acceptable salts.

5. Process according to claim 1, characterized in that it comprises reacting an alpha-acetophenone of formula:

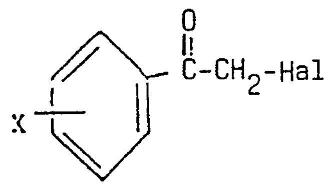

IId

wherein X is as defined above and Hal is selected from chlorine, bromine and iodine, with an aminotetraline, having its optional hydroxy substituent optionally protected, of formula IIIb as defined in claim 3, in the presence of a reducing agent, optionally separating the stereoisomers of the obtained compounds then eliminating the optionally present protecting groups; when the substituent R° of the compound thus obtained is hydrogen, optionally treating the said compound with a $C_1$-$C_4$ alkyl halogenoacetate, in the presence of sodium hydride; and after having optionally converted the carbalkoxy group in the corresponding carboxyl group by saponification, optionally converting the product thus obtained into one of its pharmaceutically acceptable salts.

6. Process according to claim 5, characterized in that the reaction is carried out also in the presence of a tertiary amine.

7. Process according to any one of claims 1, 5 or 6, characterized in that the reducing agent is added after the neutralization and the isolation of the obtained ketone.

8. Process according to any one of claims 1 to 6, characterized in that the separation of stereoisomers of formula I is carried out by fractional crystallization in an appropriate solvent or by chromatography.

9. Process according to claim 8, characterized in that the appropriate solvent is a mixture of isopropyl alcohol and ethanol.

10. Process according to any one of claims 1, 2 and 4 to 9, characterized in that the reducing agent is hydrogen in the presence of a hydrogenation catalyst.

11. Process according to any one of claims 1, 2 or 4 to 9, characterized in that the reducing agent is a metal hydride complex.

12. Process according to claim 11, characterized in that the metal hydride complex is the sodium cyanoborohydride complex.

13. Process according to any one of claims 1 to 12, characterized in that it comprises isolating the final product in the form of a salt thereof and converting said salt into the corresponding free base.

14. Process according to any one of the preceding claims, characterized in that it comprises isolating the compound of formula I in the form of a single stereoisomer or in the form of a mixture of stereoisomer.

15. Process according to any one of claims 1 to 14, characterized in that it comprises the preparations of a compound of formula I selected from the group consisting of:
the 2-[(7-hydroxy-1, 2,3, 4-tetrahydronaphth-2-yl)amino]-1-phenylethanol,
the 2-[(7-hydroxy-1,2,3,4-tetrahydronaphth-2-yl)amino]-1-(3-chlorophenyl)ethanol,
the 2-[(7-carbethoxymethoxy-1,2,3,4-tetrahydronaphth-2-yl)amino]-1-(3-chlorophenyl)ethanol,
the 2-[(7-carbethoxymethoxy-1,2,3,4-tetrahydronaphth-2-yl)amino]-1-phenylethanol or one of their pharmaceutically acceptable salts thereof.

16. Process according to any one of claims 1 to 14, characterized in that its comprises the preparation of a compound of formula I selected from:
the (1R,2'RS)-2-[(7-hydroxy-1,2,3,4 -tetrahydronaphth-2-yl)amino]-1-phenylethanol,
the (1S,2'RS)-2-[(7-hydroxy-1,2,3,4-tetrahydronaphth-2-yl)amino]-1-phenylethanol,
the (+)-(1R)-2-[(7-hydroxy-1,2,3,4-tetrahydronaphth-2-yl)amino]-1-phenylethanol,
the (+)-(1S)-2-[(7-hydroxy-1,2,3,4-tetrahydronaphth-2-yl)amino]-1-phenylethanol,
the (-)-(1R)-2-[(7-hydroxy-1,2,3,4-tetrahydronaphth-2-yl)amino]-1-phenylethanol,

the (-)-(1S)-2-[(7-hydroxy-1,2,3,4-tetrahydronaphth-2-yl)amino]-1-phenylethanol or one of their pharmaceutically acceptable salts.

**Patentansprüche** für die Vertragsstaaten: BE, CH, DE, FR, GB, IT, LI, LU, NL, SE

1. Verbindung der Formel:

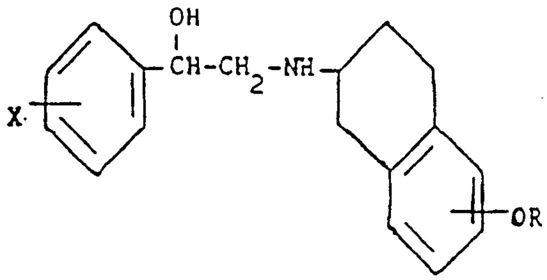

I

worin X Wasserstoff, ein Halogen, eine Trifluormethylgruppe oder eine $C_1$-$C_4$-Alkylgruppe darstellt und R für Wasserstoff oder eine nicht substituierte oder durch eine Carboxy- oder Carbalkoxygruppe, deren Alkylgruppe in $C_1$-$C_4$ steht, substituierte Methylgruppe steht, oder eines ihrer pharmazeutisch akzeptablen Salze.

2. Verbindung der Formel I nach Anspruch 1 dadurch gekennzeichnet, daß sie ausgewählt ist aus:
2-[(7-Hydroxy-1,2,3,4-tetrahydronaphth-2-yl)-amino]-1-phenylethanol,
2-[(7-Hydroxy-1,2,3,4-tetrahydronaphth-2-yl)-amino]-1-(3-chlorphenyl)-ethanol,
2-[(7-Carbethoxymethoxy-1,2,3,4-tetrahydronaphth-2-yl)-amino]-1-(3-chlorphenyl)-ethanol,
2-[(7-Carbethoxymethoxy-1,2,3,4-tetrahydronaphth-2-yl)-amino]-1-phenylethanol
oder einem ihrer pharmazeutisch akzeptablen Salze.

3. Verbindung nach einem der Ansprüche 1 oder 2 in Form eines einzigen Stereoisomeren oder in Form einer Mischung von Stereoisomeren.

4. Verbindung der Formel I nach Anspruch 1, dadurch gekennzeichnet, daß sie ausgewählt ist aus:
(1R,2'RS)-2-[(7-Hydroxy-1,2,3,4-tetrahydronaphth-2-yl)-amino]-1-phenylethanol,
(1S,2'RS)-2-[(7-Hydroxy-1,2,3,4-tetrahydronaphth-2-yl)-amino]-1-phenylethanol,
(+)-(1R)-2-[(7-Hydroxy-1,2,3,4-tetrahydronaphth-2-yl)-amino]-1-phenylethanol,
(+)-(1S)-2-[(7-Hydroxy-1,2,3,4-tetrahydronaphth-2-yl)-amino]-1-phenylethanol,
(-)-(1R)-2-[(7-Hydroxy-1,2,3,4-tetrahydronaphth-2-yl)-amino]-1-phenylethanol,
(-)-(1S)-2-[(7-Hydroxy-1,2,3,4-tetrahydronaphth-2-yl)-amino]-1-phenylethanol
oder einem ihrer pharmazeutisch akzeptablen Salze.

5. Verfahren zur Herstellung einer Verbindung nach einem der Ansprüche 1 bis 4, dadurch gekennzeichnet, daß man eine Verbindung der Formel:

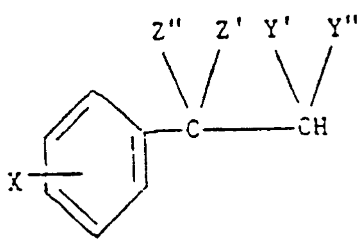

II

worin X wie oben definiert ist, eines von Z' und Z" Wasserstoff und das andere eine Hydroxygruppe ist, oder aber Z' und Z" zusammen eine Oxogruppe bilden, eines von Y' und Y" Wasserstoff und das andere ein Halogen, ausgewählt aus Chlor, Brom und Jod, oder eine Aminogruppe ist, oder aber Y' und Y" zusammen eine Oxogruppe bilden, oder Y" und Z" beide Wasserstoff sind und Y' und Z' zusammen ein Epoxidsauerstoffatom bilden, mit einem Tetralin, dessen eventueller Hydroxysubstituent gegebenenfalls geschützt ist, der Formel:

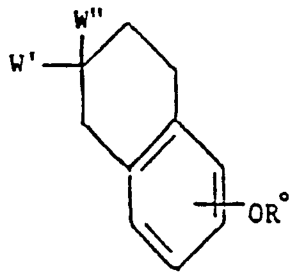

III

worin R° Wasserstoff oder Methyl ist und entweder eines von W' und W" Wasserstoff und das andere eine Aminogruppe darstellt oder W' und W" zusammen eine Oxogruppe bilden, zur Umsetzung bringt, wobei die Reaktion in Gegenwart eines Reduktionsmittels ausgeführt wird, wenn mindestens eine Oxogruppe in einer der Verbindungen II und III vorhanden ist, man gegebenenfalls die Stereoisomeren der erhaltenen Verbindungen trennt, man die gegebenenfalls vorhandenen Schutzgruppen entfernt, man, wenn der Substituent R° der so erhaltenen Verbindung Wasserstoff ist, gegebenenfalls die Verbindung mit einem $C_1$-$C_4$-Alkylhalogenacetat in Gegenwart von Natriumhydrid behandelt und man, nachdem gegebenenfalls die Carbalkoxygruppe durch Verseifung in die entsprechende Carboxylgruppe übergeführt worden ist, das so erhaltene Produkt gegebenenfalls in seine pharmazeutisch akzeptablen Salze überführt; mit der Einschränkung, daß in den Ausgangsverbindungen II und III

- wenn Y' oder Y" eine Aminogruppe ist, eines von W' und W" nicht eine Aminogruppe ist,
- wenn Y' und Y" zusammen eine Oxogruppe bilden, W' und W" zusammen nicht eine Oxogruppe bilden, und
- wenn Y' und Z' zusammen ein Epoxidsauerstoffatom bilden, W' und W" zusammen keine Oxogruppe sind.

6. Verfahren nach Anspruch 5, dadurch gekennzeichnet, daß man ein racemisches oder optisch aktives Phenylethanolamin der Formel

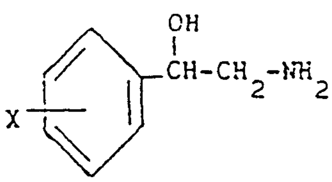

IIa

worin X wie oben definiert ist, mit einem Tetralon, dessen eventueller Hydroxysubstituent gegebenenfalls geschützt ist, der Formel

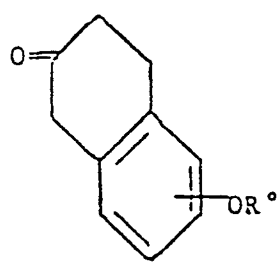

IIIa

worin R° wie oben definiert ist, in einem organischen Lösungsmittel in Gegenwart eines Reduktionsmittels zur Umsetzung bringt, man gegebenenfalls die Stereoisomeren der erhaltenen Verbindungen trennt, man dann die gegebenenfalls vorhandenen Schutzgruppen entfernt, man, wenn der Substituent R° der so erhaltenen Verbindung Wasserstoff ist, gegebenenfalls die Verbindung mit einem $C_1$-$C_4$-Alkylhalogenacetat in Gegenwart von Natriumhydrid behandelt und man, nachdem gegebenenfalls die Carbalkoxygruppe durch Verseifung in die entsprechende Carboxylgruppe übergeführt worden ist, gegebenenfalls das so erhaltene Produkt in eines seiner pharmazeutisch akzeptablen Salze überführt.

7. Verfahren nach Anspruch 5, dadurch gekennzeichnet, daß man eine Epoxidverbindung der Formel:

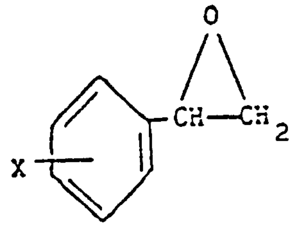

IIb

worin X wie oben definiert ist, mit einem Aminotetralin, dessen eventueller Hydroxysubstituent gegebenenfalls geschützt ist, der Formel:

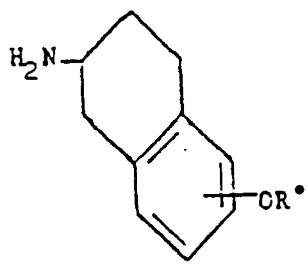

IIIb

worin R° wie oben definiert ist, zur Umsetzung bringt, man gegebenenfalls die Stereoisomeren der erhaltenen Verbindungen trennt, man dann die gegebenenfalls vorhandenen Schutzgruppen entfernt, man, wenn der Substituent R° der so erhaltenen Verbindung Wasserstoff ist, gegebenenfalls die Verbindung mit einem $C_1$-$C_4$-Alkylhalogenacetat in Gegenwart von Natriumhydrid behandelt und man, nachdem gegebenenfalls die Carbalkoxygruppe durch Verseifung in die entsprechende Carboxylgruppe übergeführt worden ist, gegebenenfalls das so erhaltene Produkt in eines seiner pharmazeutisch akzeptablen Salze überführt.

8. Verfahren nach Anspruch 5, dadurch gekennzeichnet, daß man ein Phenylglyoxal der Formel:

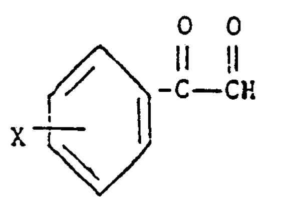

IIc

worin X wie oben definiert ist, mit einem Aminotetralin, dessen eventueller Hydroxysubstituent gegebenenfalls geschützt ist, der Formel IIIb wie in Anspruch 7 definiert in Gegenwart eines Reduktionsmittels zur Umsetzung bringt, man gegebenenfalls die Stereoisomeren der erhaltenen Verbindungen trennt, man dann die gegebenenfalls vorhandenen Schutzgruppen entfernt, man, wenn der Substituent R° der so erhaltenen Verbindung Wasserstoff ist, gegebenenfalls die Verbindung mit einem $C_1$-$C_4$-Alkylhalogenacetat in Gegenwart von Natriumhydrid behandelt und man, nachdem gegebenenfalls die Carbalkoxygruppe durch Verseifung in die entsprechende Carboxylgruppe übergeführt worden ist, gegebenenfalls das so erhaltene Produkt in eines seiner pharmazeutisch akzeptablen Salze überführt.

9. Verfahren nach Anspruch 5, dadurch gekennzeichnet, daß man ein alpha-Acetophenon der Formel:

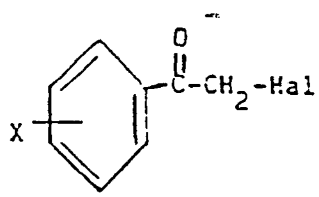

IId

worin X wie oben definiert ist und Hal ausgewählt ist aus Chlor, Brom und Jod, mit einem Aminotetralin, dessen eventueller Hydroxysubstituent gegebenenfalls geschützt ist, der Formel IIIb wie in Anspruch 7 definiert in Gegenwart eines Reduktionsmittels zur Umsetzung bringt, man gegebenenfalls die Stereoisomeren der erhaltenen Verbindungen trennt, man dann die gegebenenfalls vorhandenen Schutzgruppen entfernt, man, wenn der Substituent R° der so erhaltenen Verbindung Wasserstoff ist, gegebenenfalls die Verbindung mit einem $C_1$-$C_4$-Alkylhalogenacetat in Gegenwart von Natriumhydrid behandelt, und man, nachdem gegebenenfalls die Carbalkoxygruppe durch Verseifung in die entsprechende Carboxylgruppe übergeführt worden ist, gegebenenfalls das so erhaltene Produkt in eines seiner pharmazeutisch akzeptablen Salze überführt.

10. Verfahren nach Anspruch 9, dadurch gekennzeichnet, daß die Reaktion auch in Gegenwart eines tertiären Amins durchgeführt wird.

11. Verfahren nach einem der Ansprüche 5, 9 oder 10, dadurch gekennzeichnet, daß das Reduktionsmittel nach der Neutralisierung und Isolierung des erhaltenen Ketons zugegeben wird.

12. Verfahren nach einem der Ansprüche 5 bis 11, dadurch gekennzeichnet, daß die Trennung der Stereoisomeren der Formel I durch fraktionierte Kristallisation in einem geeigneten Lösungsmittel oder durch Chromatografie erfolgt.

13. Verfahren nach Anspruch 12, dadurch gekennzeichnet, daß das geeignete Lösungsmittel eine Mischung aus Isopropanol und Ethanol ist.

14. Verfahren nach einem der Ansprüche 5, 6, 8 bis 13, dadurch gekennzeichnet, daß das Reduktionsmittel Wasserstoff in Gegenwart eines Hydrierungskatalysators ist.

15. Verfahren nach einem der Ansprüche 5, 6, 8 bis 13, dadurch gekennzeichnet, daß das Reduktionsmittel ein Metallhydridkomplex ist.

16. Verfahren nach Anspruch 15, dadurch gekennzeichnet, daß der Metallhydridkomplex der Komplex Cyano-Natriumborhydrid ist.

17. Verfahren nach einem der Ansprüche 5 bis 16, dadurch gekennzeichnet, daß man das Endprodukt in Form eines seiner Salze isoliert und man das Salz in die entsprechende freie Base überführt.

18. Pharmazeutische Zusammensetzung, die als Wirkstoff eine Verbindung nach einem der Ansprüche 1 bis 4 enthält.

19. Pharmazeutische Zusammensetzung nach Anspruch 18 in Dosiseinheitsform, in der der Wirkstoff mit einem pharmazeutischen Exzipienten vermischt ist.

20. Pharmazeutische Zusammensetzung nach einem der Ansprüche 18 oder 19, enthaltend 0,1 bis 500 mg Wirkstoff.

**Patentansprüche** für den Vertragsstaat: AT

1. Verfahren zur Herstellung von neuen Phenylethanolaminotetralinen der Formel:

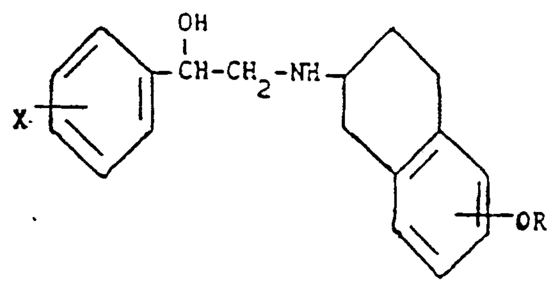

I

worin X Wasserstoff, ein Halogen, eine Trifluormethylgruppe oder eine $C_1$-$C_4$-Alkylgruppe darstellt und R für Wasserstoff oder eine nicht substituierte oder durch eine Carboxy- oder Carbalkoxygruppe, deren Alkylgruppe in $C_1$-$C_4$ steht, substituierte Methylgruppe steht, oder ihren pharmazeutisch akzeptablen Salzen, dadurch gekennzeichnet, daß man eine Verbindung der Formel:

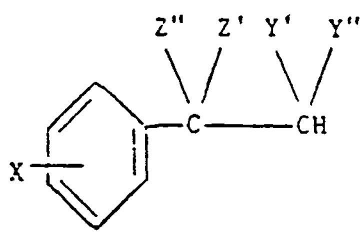

II

worin X wie oben definiert ist, eines von Z' und Z" Wasserstoff und das andere eine Hydroxygruppe ist, oder aber Z' und Z" zusammen eine Oxogruppe bilden, eines von Y' und Y" Wasserstoff und das andere ein Halogen, ausgewählt aus Chlor, Brom und Jod, oder eine Aminogruppe ist, oder aber Y' und Y" zusammen eine Oxogruppe bilden, oder Y" und Z" beide Wasserstoff sind und Y' und Z' zusammen ein Epoxidsauerstoffatom bilden, mit einem Tetralin, dessen eventueller Hydroxysubstituent gegebenenfalls geschützt ist, der Formel:

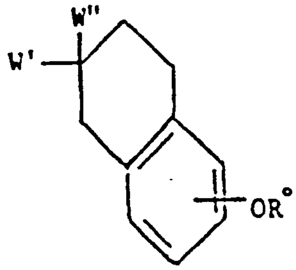

III

worin R° Wasserstoff oder Methyl ist und entweder eines von W' und W" Wasserstoff und das andere eine Aminogruppe darstellt oder W' und W" zusammen eine Oxogruppe bilden, zur Umsetzung bringt, wobei die Reaktion in Gegenwart eines Reduktionsmittels ausgeführt wird, wenn mindestens eine Oxogruppe in einer der Verbindungen II und III vorhanden ist, man gegebenenfalls die Stereoisomeren der erhaltenen Verbindungen trennt, man die gegebenenfalls vorhandenen Schutzgruppen entfernt, man, wenn der Substituent R° der so erhaltenen Verbindung Wasserstoff ist, gegebenenfalls die Verbindung mit einem $C_1$-$C_4$-Alkylhalogenacetat in Gegenwart von Natriumhydrid behandelt und man, nachdem gegebenenfalls die Carbalkoxygruppe durch Verseifung in die entsprechende Carboxylgruppe übergeführt worden ist, das so erhaltene Produkt gegebenenfalls in seine pharmazeutisch akzeptablen Salze überführt; mit der Einschränkung, daß in den Ausgangsverbindungen II und III

- wenn Y' oder Y" eine Aminogruppe ist, eines von W' und W" nicht eine Aminogruppe ist,
- wenn Y' und Y" zusammen eine Oxogruppe bilden, W' und W" zusammen nicht eine Oxogruppe bilden, und
- wenn Y' und Z' zusammen ein Epoxidsauerstoffatom bilden, W' und W" zusammen keine Oxogruppe sind.

2. Verfahren nach Anspruch 1, dadurch gekennzeichnet, daß man ein racemisches oder optisch aktives Phenylethanolamin der Formel:

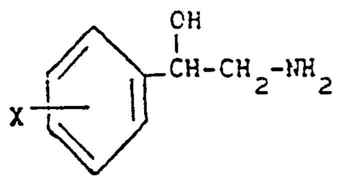

IIa

worin X wie oben definiert ist, mit einem Tetralon, dessen eventueller Hydroxysubstituent gegebenenfalls geschützt ist, der Formel:

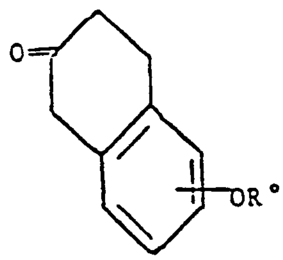

IIIa

worin R° wie oben definiert ist, in einem organischen Lösungsmittel in Gegenwart eines Reduktionsmittels zur Umsetzung bringt, man gegebenenfalls die Stereoisomeren der erhaltenen Verbindungen trennt, man dann die gegebenenfalls vorhandenen Schutzgruppen entfernt, man, wenn der Substituent R° der so erhaltenen Verbindung Wasserstoff ist, gegebenenfalls die Verbindung mit einem $C_1$-$C_4$-Alkylhalogenacetat in Gegenwart von Natriumhydrid behandelt und man, nachdem gegebenenfalls die Carbalkoxygruppe durch Verseifung in die entsprechende Carboxylgruppe übergeführt worden ist, gegebenenfalls das so erhaltene Produkt in eines seiner pharmazeutisch akzeptablen Salze überführt.

3. Verfahren nach Anspruch 1, dadurch gekennzeichnet, daß man eine Epoxidverbindung der Formel:

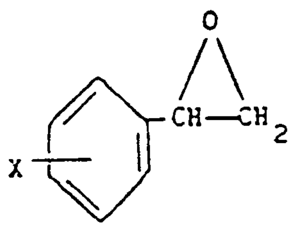

IIb

worin X wie oben definiert ist, mit einem Aminotetralin, dessen eventueller Hydroxysubstituent gegebenenfalls geschützt ist, der Formel:

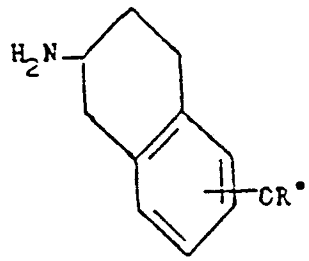

IIIb

worin R° wie oben definiert ist, zur Umsetzung bringt, man gegebenenfalls die Stereoisomeren der erhaltenen Verbindungen trennt, man dann die gegebenenfalls vorhandenen Schutzgruppen entfernt, man, wenn der Substituent R° der so erhaltenen Verbindung Wasserstoff ist, gegebenenfalls die Verbindung mit einem $C_1$-$C_4$-Alkylhalogenacetat in Gegenwart von Natriumhydrid behandelt und man, nachdem gegebenenfalls die Carbalkoxygruppe durch Verseifung in die entsprechende Carboxylgruppe übergeführt worden ist, gegebenenfalls das so erhaltene Produkt in eines seiner pharmazeutisch akzeptablen Salze überführt.

4. Verfahren nach Anspruch 1, dadurch gekennzeichnet, daß man ein Phenylglyoxal der Formel:

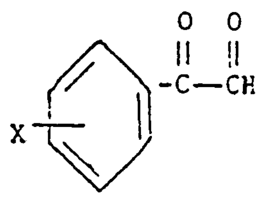

IIc

worin X wie oben definiert ist, mit einem Aminotetralin, dessen eventueller Hydroxysubstituent gegebenenfalls geschützt ist, der Formel IIIb wie in Anspruch 3 definiert in Gegenwart eines Reduktionsmittels zur Umsetzung bringt, man gegebenenfalls die Stereoisomeren der erhaltenen Verbindungen trennt, man dann die gegebenenfalls vorhandenen Schutzgruppen entfernt, man, wenn der Substituent R° der so erhaltenen Verbindung Wasserstoff ist, gegebenenfalls die Verbindung mit einem $C_1$-$C_4$-Alkylhalogenacetat in Gegenwart von Natriumhydrid behandelt und man, nachdem gegebenenfalls die Carbalkoxygruppe durch Verseifung in die entsprechende Carboxylgruppe übergeführt worden ist, gegebenenfalls das so erhaltene Produkt in eines seiner pharmazeutisch akzeptablen Salze überführt.

5. Verfahren nach Anspruch 1, dadurch gekennzeichnet, daß man ein alpha-Acetophenon der Formel:

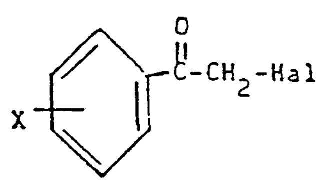

IId

worin X wie oben definiert ist und Hal ausgewählt ist aus Chlor, Brom und Jod, mit einem Aminotetralin, dessen eventueller Hydroxysubstituent gegebenenfalls geschützt ist, der Formel IIIb wie in Anspruch 3 definiert in Gegenwart eines Reduktionsmittels zur Umsetzung bringt, man gegebenenfalls die Stereoisomeren der erhaltenen Verbindungen trennt, man dann die gegebenenfalls vorhandenen Schutzgruppen entfernt, man, wenn der Substituent R° der so erhaltenen Verbindung Wasserstoff ist, gegebenenfalls die Verbindung mit einem $C_1$-$C_4$-Alkylhalogenacetat in Gegenwart von Natriumhydrid behandelt, und man, nachdem gegebenenfalls die Carbalkoxygruppe durch Verseifung in die entsprechende Carboxylgruppe übergeführt worden ist, gegebenenfalls das so erhaltene Produkt in eines seiner pharmazeutisch akzeptablen Salze überführt.

6. Verfahren nach Anspruch 5, dadurch gekennzeichnet, daß die Reaktion auch in Gegenwart eines tertiären Amins durchgeführt wird.

7. Verfahren nach einem der Ansprüche 1, 5 oder 6, dadurch gekennzeichnet, daß das Reduktionsmittel nach der Neutralisierung und Isolierung des erhaltenen Ketons zugegeben wird.

8. Verfahren nach einem der Ansprüche 1 bis 6, dadurch gekennzeichnet, daß die Trennung der Stereoisomeren der Formel I durch fraktionierte Kristallisation in einem geeigneten Lösungsmittel oder durch Chromatografie erfolgt.

9. Verfahren nach Anspruch 8 , dadurch gekennzeichnet, daß das geeignete Lösungsmittel eine Mischung aus Isopropanol und Ethanol ist.

10. Verfahren nach einem der Ansprüche 1, 2 oder 4 bis 9, dadurch gekennzeichnet, daß das Reduktionsmittel Wasserstoff in Gegenwart eines Hydrierungskatalysators ist.

11. Verfahren nach einem der Ansprüche 1, 2 oder 4 bis 9, dadurch gekennzeichnet, daß das Reduktionsmittel ein Metallhydridkomplex ist.

12. Verfahren nach Anspruch 11, dadurch gekennzeichnet, daß der Metallhydridkomplex der Komplex Cyano-Natriumborhydrid ist.

13. Verfahren nach einem der Ansprüche 1 bis 12, dadurch gekennzeichnet, daß man das Endprodukt in Form eines seiner Salze isoliert und man das Salz in die entsprechende freie Base überführt.

14. Verfahren nach einem der vorhergehenden Ansprüche, dadurch gekennzeichnet, daß man die Verbindung der Formel I in Form eines einzigen Stereoisomeren oder in Form einer Mischung von Stereoisomeren isoliert.

15. Verfahren nach einem der Ansprüche 1 bis 14, dadurch gekennzeichnet, daß man eine Verbindung der Formel I herstellt, die ausgewählt ist aus:
2-[(7-Hydroxy-1,2,3,4-tetrahydronaphth-2-yl)-amino]-1-phenylethanol,
2-[(7-Hydroxy-1,2,3,4-tetrahydronaphth-2-yl)-amino]-1-(3-chlorphenyl)-ethanol,
2-[(7-Carbethoxymethoxy-1,2,3,4-tetrahydronaphth-2-yl)-amino]-1-(3-chlorphenyl)-ethanol,
2-[(7-Carbethoxymethoxy-1,2,3,4-tetrahydronaphth-2-yl)-amino]-1-phenylethanol
oder einem ihrer pharmazeutisch akzeptablen Salze.

16. Verfahren nach einem der Ansprüche 1 bis 14, dadurch gekennzeichnet, daß man eine Verbindung der Formel I herstellt, die ausgewählt ist aus:
(1R,2'RS)-2-[(7-Hydroxy-1,2,3,4-tetrahydronaphth-2-yl)-amino]-1-phenylethanol,
(1S,2'RS)-2-[(7-Hydroxy-1,2,3,4-tetrahydronaphth-2-yl)-amino]-1-phenylethanol,
(+)-(1R)-2-[(7-Hydroxy-1,2,3,4-tetrahydronaphth-2-yl)-amino]-1-phenylethanol,
(+)-(1S)-2-[(7-Hydroxy-1,2,3,4-tetrahydronaphth-2-yl)-amino]-1-phenylethanol,
(-)-(1R)-2-[(7-Hydroxy-1,2,3,4-tetrahydronaphth-2-yl)-amino]-1-phenylethanol,
(-)-(1S)-2-[(7-Hydroxy-1,2,3,4-tetrahydronaphth-2-yl)-amino]-1-phenylethanol
oder einem ihrer pharmazeutisch akzeptablen Salze.